# EUROPEAN PATENT SPECIFICATION

(11) **EP 4 121 415 B1**
(45) Date of publication and mention of the grant of the patent: **07.02.2024**
(21) Application number: 20732479.9
(22) Date of filing: 02.06.2020
(51) Int. Cl.: C07D 213/61, A01N 47/30, A01N 47/36, C07C 275/32, C07C 275/34, C07D 213/63, A61P 21/00

(54) **INHIBITORS OF CYTOKININ OXIDASE DERIVED FROM 1-[2-(HYDROXYALKYL)PHENYL]-3-YLUREA, USE THEREOF AND PREPARATIONS CONTAINING THESE DERIVATIVES**
INHIBITOREN DER CYTOKININOXIDASE AUS 1-[2-(HYDROXYALKYL)PHENYL)-3-YL HARNSTOFF, IHRE VERWENDUNG UND DIESE DERIVATE ENTHALTENDE ZUBEREITUNGEN
INHIBITEURS DE LA CYTOKININE OXYDASE DÉRIVÉS DE 1-[2-(HYDROXYALKYL)PHÉNYL]-3-YL-URÉE, LEUR UTILISATION ET PRÉPARATIONS CONTENANT CES DÉRIVÉS

(30) Priority: 16.03.2020 CZ 20200144
(43) Date of publication of application: 25.01.2023
(73) Proprietor: Univerzita Palackého v Olomouci, 779 00 Olomouc (CZ)
(72) Inventor: NISLER, Jaroslav, 14000 Praha 4 (CZ); KOPECNY, David, 79604 Prostejov (CZ); SPICHAL, Lukas, 77900 Bystrocice (CZ); STRNAD, Miroslav, 77900 Olomouc (CZ)
(74) Representative: Harber IP s.r.o.
(86) International application number: PCT/CZ2020/050038
(87) International publication number: WO 2021/185391

(56) References cited:
- CN-A- 107 892 598
- CN-B- 106 831 494
- JP-A- 2003 192 659
- KOPECNY D ET AL: "Phenyl- and benzylurea cytokinins as competitive inhibitors of cytokinin oxidase/dehydrogenase: A structural study", BIOCHIMIE, MASSON, PARIS, FR, vol. 92, no. 8, 1 August 2010 (2010-08-01) , pages 1052-1062, XP027141295, ISSN: 0300-9084 [retrieved on 2010-05-15]
- DATABASE REGISTRY [Online] CHEMICAL ABSTRACTS SERVICE, COLUMBUS, OHIO, US; 3 June 2011 (2011-06-03), XP002800482, Database accession no. 1304973-73-6
- DATABASE REGISTRY [Online] CHEMICAL ABSTRACTS SERVICE, COLUMBUS, OHIO, US; 4 April 2001 (2001-04-04), XP002800483, Database accession no. 329980-76-9
- DATABASE REGISTRY [Online] CHEMICAL ABSTRACTS SERVICE, COLUMBUS, OHIO, US; 23 June 2016 (2016-06-23), XP002800484, Database accession no. 1937764-06-1
- DATABASE REGISTRY [Online] CHEMICAL ABSTRACTS SERVICE, COLUMBUS, OHIO, US; 21 April 2011 (2011-04-21), XP002800485, Database accession no. 1283438-02-7
- DATABASE REGISTRY [Online] CHEMICAL ABSTRACTS SERVICE, COLUMBUS, OHIO, US; 15 July 2004 (2004-07-15), XP002800486, Database accession no. 710290-51-0

## Description

### Technical Field

The invention relates to substituted 1-[2-(hydroxyalkyl)phenyl]-3-ylurea derivatives, their use as stimulators of plant growth and development in optimal and stress conditions, and preparations containing these derivatives.

### Background Art

Plant hormones cytokinins regulate plant growth and development. Cytokinins have been extensively tested in field conditions and have been shown to improve plant development and to increase the yield of agriculturally important crops. A positive effect on both parameters was observed in both optimal and stress conditions (summarized in Koprna R. et al., 2016, Bioorg. Med. Chem. 24, 484-492).

Cytokinin oxidase/dehydrogenase (CKX, EC 1.5.99.12) is, an enzyme, that degrades cytokinins in all plants. Thus, inhibition of this enzyme leads to better development and higher yields of various agriculturally important crops. For example, it was observed an increase in tillering and total seed yield in rice (Ashikari M. et al., 2005, Science 309, 741-745; Yeh S.Y. et al., 2015, Rice (NY) 8, 36-49). In the model plant *Arabidopsis thaliana,* inhibition of CKX also leads to an increase in the number of flowers and, subsequently, in the number of siliques, which has led to an increase in overall yield (Bartrina I. et al., 2011, The Plant Cell 23, 69-80). Nagar et al. (2015, Bioscan 10, 67-72) showed that cytokinin application increased seed yield in drought-tolerant and drought-sensitive wheat varieties, under optimal and water-limited conditions. Inhibition of the CKX enzyme clearly leads to an increase in the content of endogenous cytokinins in plants, which has a positive effect on their development under adverse conditions.

Several groups of compounds possessing CKX inhibitory activity have been developed in the past. These include in particular CPPU - 1-(2-chloropyridin-4-yl)-3phenylurea, DCPPU - 1-(2,6-dichloropyridin-4-yl)-3phenylurea and their derivatives (Kopečný D. et al., 2010, Biochimie 92, 1052-62). However, these substances have a low inhibitory potency to the CKX enzyme. For this reason, there is a need to provide new groups of compounds with a higher inhibitory strength with this enzyme. Other structurally similar substances are also disclosed in patent documents CN106831494B and CN107892598A. The development of new CKX inhibitors is desired and highly appreciated by the agrochemical industry.

### Disclosure of the Invention

We have found that CPPU, DCPPU and similar diphenyl urea derivatives have significantly increased CKX inhibitory activity when bearing the hydroxymethyl or 2-hydroxyethyl group on an empty phenyl ring in the *ortho* position. Some derivatives bearing a 2-hydroxyethyl group have already been prepared. However, these derivatives are only known for pharmaceutical use as painkillers (JP 2003192659 and 20030709). None of these substances have ever been described for use on plants. These substances increase the yield and resistance of plants to stress.

The object of the present invention is the compound of the general formula (I), 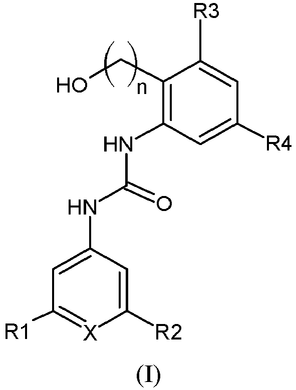 wherein
n is 1 or 2
X is nitrogen atom (N) or carbon atom (C);
R1 is selected from the group consisting of halogen, methoxy group, trifluoromethoxy group, methylsulphanyl group or trifluoromethylsulphanyl group;
R2 is selected from the group consisting of hydrogen, halogen, amino group, C₁-C₅ alkyl, C₂-C₅ alkenyl, C₂-C₅ alkynyl, and C₁-C₅ alkylamino group, wherein C₁-C₅ alkyl, C₂-C₅ alkenyl, C₂-C₅ alkynyl and C-C₅ alkylamino group may optionally be substituted with hydroxy and/or amino group;
R3 and R4 are independently hydrogen or halogen;
with the proviso that when X is nitrogen, then R2 is hydrogen or halogen;
and with the proviso that 1-(2,6-dichloropyridin-4-yl)-3-[2-(2-hydroxyethyl)phenyl]urea, 1-[2-(2-hydroxyethyl)phenyl]-3-(3-(trifluoromethoxy)phenyl)urea, 1-[2-(2-hydroxyethyl)phenyl]-3-(3-methoxyphenyl)urea, 1-(3,5-dichlorophenyl)-3-[2-(2-hydroxyethyl)phenyl]urea, 1-(3,5-difluorophenyl)-3-[2-(2-hydroxyethyl)phenyl]urea, 1-(3-fluorophenyl)-3-[2-(2-hydroxyethyl)-phenyl]urea, 1-(3-chlorophenyl)-3-[2-(2-hydroxyethyl)phenyl]urea, 1-(2-hydroxymethylphenyl)-3-(3-methoxyphenyl)urea, 1-(3,5-dichlorophenyl)-3-(2-hydroxymethylphenyl)urea, 1-(3-fluorophenyl)-3-(2-hydroxymethylphenyl)urea, and 1-(3-chlorophenyl)-3-(2-hydroxymethylphenyl)urea are excluded.

The generic substituent groups have meanings as defined herein below, wherein
alkyl denotes branched or linear alkyl group;
(C₁-C₅) alkyl denotes branched or linear alkyl group with 1 up to 5 carbon atoms;
alkenyl denotes branched or linear hydrocarbon chain with at least one double bond;
alkynyl denotes branched or linear hydrocarbon chain with at least one triple bond;
halogen is selected from the group comprising fluorine, chlorine, bromine and iodine atom;
hydroxy denotes the group -OH;
alkyloxy denotes the group -O-alkyl, preferably, alkyloxy is methoxy or ethyloxy group; trifluoroalkyloxy group denotes the group -O-trifluoroalkyl, preferably, it is trifluoromethoxy group - OCF₃;
methylsulphanyl group denotes to -SCH₃;
trifluoromethylsulphanyl group denotes to -SCF₃;
alkylthio group denotes to -S-alkyl, preferably, alkylthio is methylthio or ethylthio.

When the compounds of this invention contain a chiral centre, then all enantiomers, mixtures of enantiomers and racemates fall within the framework of the present invention. The present invention further includes the compounds of general formula (I) in the form of salts with alkali metals, ammonium or amines, as well as in the form of addition salts with acids.

In one preferred embodiment n = 1 (hydroxymethyl).

In one preferred embodiment R1 is selected from the group consisting of fluorine, chlorine, bromine, methoxy group, trifluoromethoxy group, methylsulphanyl group and trifluoromethylsulphanyl group.

In one preferred embodiment R2 is selected from the group consisting of hydrogen, fluorine, chlorine, bromine, amino group, 2-aminoethylamino group and 2-hydroxyethylamino group.

In one preferred embodiment R3 and R4 are independently selected from the group consisting of hydrogen, fluorine, chlorine, and bromine. Preferably, R4 is fluorine, chlorine or bromine and R3 is hydrogen.

In one preferred embodiment is n = 1, R1 is selected from the group consisting of fluorine, chlorine, bromine, methoxy group, trifluoromethoxy group, methylsulphanyl group and trifluoromethylsulphanyl group; R2 is selected from the group consisting of hydrogen, fluorine, chlorine, bromine, amino group, 2-aminoethylamino group and 2-hydroxyethylamino group; R3 and R4 are independently hydrogen, fluorine, chlorine, or bromine atom.

In one preferred embodiment, X = N, n = 1 and the compound of the general formula (I) is selected from the group comprising:
**R1 is F, R2 is H:** 1-(2-fluoropyridin-4-yl)-3-(2-hydroxymethylphenyl)urea, 1-(3-fluoro-2-hydroxymethylphenyl)-3-(2-fluoropyridin-4-yl)urea, 1-(3-chloro-2-hydroxymethylphenyl)-3-(2-fluoropyridin-4-yl)urea, 1-(3 -bromo-2-hydroxymethylphenyl)-3 -(2-fluoropyridin-4-yl)urea, 1-(5-fluoro-2-hydroxymethylphenyl)-3-(2-fluoropyridin-4-yl)urea, 1-(5-chloro-2-hydroxymethylphenyl)-3-(2-fluoropyridin-4-yl)urea, 1-(5-bromo-2-hydroxymethylphenyl)-3-(2-fluoropyridin-4-yl)urea, 1-(3,5-difluoro-2-hydroxymethylphenyl)-3-(2-fluoropyridin-4-yl)urea, 1-(3,5 -dichloro-2-hydroxymethylphenyl)-3 -(2-fluoropyridin-4-yl)urea, 1-(3,5-dibromo-2-hydroxymethylphenyl)-3-(2-fluoropyridin-4-yl)urea,
**R1 is F, R2 is F:** 1-(2,6-difluoropyridin-4-yl)-3-(2-hydroxymethylphenyl)urea, 1-(3-fluoro-2-hydroxymethylphenyl)-3-(2,6-difluoropyridin-4-yl)urea, 1-(3-chloro-2-hydroxymethylphenyl)-3-(2,6-difluoropyridin-4-yl)urea, 1-(3-bromo-2-hydroxymethylphenyl)-3-(2,6-difluoropyridin-4-yl)urea, 1-(5-fluoro-2-hydroxymethylphenyl)-3-(2,6-difluoropyridin-4-yl)urea, 1-(5-chloro-2-hydroxymethylphenyl)-3-(2,6-difluoropyridin-4-yl)urea, 1-(5-bromo-2-hydroxymethylphenyl)-3-(2,6-difluoropyridin-4-yl)urea, 1-(3,5-difluoro-2-hydroxymethylphenyl)-3-(2,6-difluoropyridin-4-yl)urea, 1-(3,5-dichloro-2-hydroxymethylphenyl)-3-(2,6-difluoropyridin-4-yl)urea, 1-(3,5-dibromo-2-hydroxymethylphenyl)-3-(2,6-difluoropyridin-4-yl)urea,
**R1 is Cl, R2 is H:** 1-(2-chloropyridin-4-yl)-3-(2-hydroxymethylphenyl)urea, 1-(2-chloropyridin-4-yl)-3-(3-fluoro-2-hydroxymethylphenyl)urea, 1-(3-chloro-2-hydroxymethylphenyl)-3-(2-chloropyridin-4-yl)urea, 1-(3-bromo-2-hydroxymethylphenyl)-3-(2-chloropyridin-4-yl)urea, 1-(2-chloropyridin-4-yl)-3-(5-fluoro-2-hydroxymethylphenyl)urea, 1-(5-chloro-2-hydroxymethylphenyl)-3-(2-chloropyridin-4-yl)urea, 1-(5-bromo-2-hydroxymethylphenyl)-3-(2-chloropyridin-4-yl)urea, 1-(2-chloropyridin-4-yl)-3-(3,5-difluoro-2-hydroxymethylphenyl)urea, 1-(3,5-dichloro-2-hydroxymethylphenyl)-3-(2-chloropyridin-4-yl)urea, 1-(3,5-dibromo-2-hydroxymethylphenyl)-3-(2-chloropyridin-4-yl)urea,
**R1 is Cl, R2 is Cl:** 1-(2,6-dichloropyridin-4-yl)-3-(2-hydroxymethylphenyl)urea, 1-(2,6-dichloropyridin-4-yl)-3-(3-fluoro-2-hydroxymethylphenyl)urea, 1-(3-chloro-2-hydroxymethylphenyl)-3-(2,6-dichloropyridin-4-yl)urea, 1-(3-bromo-2-hydroxymethylphenyl)-3-(2,6-dichloropyridin-4-yl)urea, 1-(2,6-dichloropyridin-4-yl-3- (5-fluoro-2-hydroxymethylphenyl)urea, 1-(5-chloro-2-hydroxymethylphenyl)-3-(2,6-dichloropyridin-4-yl)urea, 1-(5-bromo-2-hydroxymethylphenyl)-3-(2,6-dichloropyridin-4-yl)urea, 1-(2,6-dichloropyridin-4-yl)-3-(3,5-difluoro-2-hydroxymethylphenyl)urea, 1-(3,5-dichloro-2-hydroxymethylphenyl)-3-(2,6-dichloropyridin-4-yl)urea, 1-(3,5-dibromo-2-hydroxymethylphenyl)-3-(2,6-dichloropyridin-4-yl)urea,
**R1 is Br, R2 is H:** 1-(2-bromopyridin-4-yl)-3-(2-hydroxymethylphenyl)urea, 1-(2-bromopyridin-4-yl)-3-(3-fluoro-2-hydroxymethylphenyl)urea, 1-(2-bromopyridin-4-yl)-3-(3-chloro-2-hydroxymethylphenyl)urea, 1-(3-bromo-2-hydroxymethylphenyl)-3-(2-bromopyridin-4-yl)urea, 1-(2-bromopyridin-4-yl)-3-(5-fluoro-2-hydroxymethylphenyl)urea, 1-(2-bromopyridin-4-yl)-3-(5-chloro-2-hydroxymethylphenyl)urea, 1-(5-bromo-2-hydroxymethylphenyl)-3-(2-bromopyridin-4-yl)urea, 1-(2-bromopyridin-4-yl)-3-(3,5-difluoro-2-hydroxy-methylphenyl)urea, 1-(2-bromopyridin-4-yl)-3-(3,5-dichloro-2-hydroxymethylphenyl)urea, 1-(3,5-dibromo-2-hydroxymethylphenyl)-3-(2-bromopyridin-4-yl)urea,
**R1 is OCH₃, R2 is H:** 1-(2-methoxypyridin-4-yl)-3-(2-hydroxymethylphenyl)urea, 1-(3-fluoro-2-hydroxymethylphenyl)-3-(2-methoxypyridin-4-yl)urea, 1-(3-chloro-2-hydroxymethylphenyl)-3-(2-methoxypyridin-4-yl)urea, 1-(3-bromo-2-hydroxymethylphenyl)-3-(2-methoxypyridin-4-yl)urea, 1-(5-fluoro-2-hydroxymethylphenyl)-3-(2-methoxypyridin-4-yl)urea, 1-(5-chloro-2-hydroxymethylphenyl)-3-(2-methoxypyridin-4-yl)urea, 1-(5-bromo-2-hydroxy-methylphenyl)-3-(2-methoxypyridin-4-yl)urea, 1-(3,5-difluoro-2-hydroxymethylphenyl)-3-(2-methoxypyridin-4-yl)urea, 1-(3,5-dichloro-2-hydroxymethylphenyl)-3-(2-methoxypyridin-4-yl)urea, 1-(3,5-dibromo-2-hydroxymethylphenyl)-3-(2-methoxypyridin-4-yl)urea,

In one preferred embodiment, X = C, n = 1 and the compound of the general formula (I) is selected from the group comprising:
**R1 is SCF₃, R2 is H:** 1-(2-hydroxymethylphenyl)-3-(3-trifluoromethylsulphanylphenyl)urea, 1-(3-fluoro-2-hydroxymethylphenyl)-3-(3-trifluoromethylsulphanylphenyl)urea, 1-(3-chloro-2-hydroxymethylphenyl)-3-(3-trifluoromethylsulphanylphenyl)urea, 1-(3-bromo-2-hydroxymethylphenyl)-3-(3 -trifluoromethyl sulphanylphenyl)urea, 1-(5-fluoro-2-hydroxymethylphenyl)-3 -(3 -trifluoromethyl sulphanylphenyl)urea, 1-(5 -chloro-2-hydroxymethylphenyl)-3 -(3 -trifluoromethylsulphanylphenyl)urea, 1-(5-bromo-2-hydroxymethylphenyl)-3-(3-trifluoromethyl-sulphanylphenyl)urea, 1-(3,5-difluoro-2-hydroxymethylphenyl)-3-(3-trifluoromethylsulphanylphenyl)urea, 1-(3,5-dichloro-2-hydroxymethylphenyl)-3-(3-trifluoromethylsulphanylphenyl)urea, 1-(3,5-dibromo-2-hydroxymethylphenyl)-3-(3-trifluoromethylsulphanylphenyl)urea,
**R1 is SCH₃, R2 is H:** 1-(2-hydroxymethylphenyl)-3-(3-methylsulphanylphenyl)urea, 1-(3-fluoro-2-hydroxymethylphenyl)-3-(3-methylsulphanylphenyl)urea, 1-(3-chloro-2-hydroxymethylphenyl)-3-(3-methylsulphanylphenyl)urea, 1-(3-bromo-2-hydroxymethylphenyl)-3-(3-methylsulphanylphenyl)urea, 1-(5-fluoro-2-hydroxymethylphenyl)-3-(3-methylsulphanylphenyl)urea, 1-(5-chloro-2-hydroxymethylphenyl)-3-(3-methylsulphanylphenyl)urea, 1-(5-bromo-2-hydroxy-methylphenyl)-3-(3-methylsulphanylphenyl)urea, 1-(3,5-difluoro-2-hydroxymethylphenyl)-3-(3-methylsulphanylphenyl)urea, 1-(3,5-dichloro-2-hydroxymethylphenyl)-3-(3-methylsulphanylphenyl)urea, 1-(3,5-dibromo-2-hydroxymethylphenyl)-3-(3-methylsulphanylphenyl)urea,
**R1 is OCF₃, R2 is H:** 1-(2-hydroxymethylphenyl)-3-(3-trifluoromethoxyphenyl)urea, 1-(3-fluoro-2-hydroxymethylphenyl)-3-(3-trifluoromethoxyphenyl)urea, 1-(3-chloro-2-hydroxymethylphenyl)-3-(3-trifluoromethoxyphenyl)urea, 1-(3-bromo-2-hydroxymethylphenyl)-3-(3-trifluoro-methoxyphenyl)urea, 1-(5-fluoro-2-hydroxymethylphenyl)-3-(3-trifluoromethoxyphenyl)urea, 1-(5-chloro-2-hydroxymethylphenyl)-3-(3-trifluoromethoxyphenyl)urea, 1-(5-bromo-2-hydroxymethylphenyl)-3-(3-trifluoromethoxyphenyl)urea, 1-(3,5-difluoro-2-hydroxymethylphenyl)-3-(3-trifluoromethoxyphenyl)urea, 1-(3,5-dichloro-2-hydroxymethylphenyl)-3-(3-trifluoromethoxyphenyl)urea, 1-(3,5-dibromo-2-hydroxymethylphenyl)-3-(3-trifluoromethoxyphenyl)urea,
**R1 is OCF₃, R2 is F:** 1-(3-fluoro-5-trifluoromethoxyphenyl)-3-(2-hydroxymethylphenyl)urea, 1-(3-fluoro-2-hydroxymethylphenyl)-3-(3-fluoro-5-trifluoromethoxyphenyl)urea, 1-(3-chloro-2-hydroxymethylphenyl)-3-(3-fluoro-5-trifluoromethoxyphenyl)urea, 1-(3-bromo-2-hydroxymethylphenyl)-3-(3-fluoro-5-trifluoromethoxyphenyl)urea, 1-(5-fluoro-2-hydroxymethylphenyl)-3-(3-fluoro-5-trifluoromethoxyphenyl)urea, 1-(5-chloro-2-hydroxymethylphenyl)-3-(3-fluoro-5-trifluoromethoxyphenyl)urea, 1-(5-bromo-2-hydroxymethylphenyl)-3-(3-fluoro-5-trifluoromethoxyphenyl)urea, 1-(3,5-difluoro-2-hydroxymethylphenyl)-3-(3-fluoro-5-trifluoromethoxyphenyl)urea, 1-(3,5-dichloro-2-hydroxymethylphenyl)-3-(3-fluoro-5-trifluoromethoxyphenyl)urea, 1-(3,5-dibromo-2-hydroxymethylphenyl)-3-(3-fluoro-5-trifluoromethoxyphenyl)urea,
**R1 is OCF₃, R2 is Cl:** 1-(3-chloro-5-trifluoromethoxyphenyl)-3-(2-hydroxymethylphenyl)urea, 1-(3-chloro-5-trifluoromethoxyphenyl)-3-(3-fluoro-2-hydroxymethylphenyl)urea, 1-(3-chloro-2-hydroxymethylphenyl)-3-(3-chloro-5-trifluoromethoxyphenyl)urea, 1-(3-bromo-2-hydroxymethylphenyl)-3-(3-chloro-5-trifluoromethoxyphenyl)urea, 1-(5-fluoro-2-hydroxymethylphenyl)-3-(3-chloro-5-trifluoromethoxyphenyl)urea, 1-(5-chloro-2-hydroxymethylphenyl)-3-(3-chloro-5-trifluoromethoxyphenyl)urea, 1-(5-bromo-2-hydroxymethylphenyl)-3-(3-chloro-5-trifluoromethoxyphenyl)urea, 1-(3,5-difluoro-2-hydroxymethylphenyl)-3-(3-chloro-5-trifluoro-methoxyphenyl)urea, 1-(3,5-dichloro-2-hydroxymethylphenyl)-3-(3-chloro-5-trifluoromethoxyphenyl)urea, 1-(3,5-dibromo-2-hydroxymethylphenyl)-3-(3-chloro-5-trifluoromethoxyphenyl)urea,
**R1 is OCF₃, R2 is Br:** 1-(3-bromo-5-trifluoromethoxyphenyl)-3-(2-hydroxymethylphenyl)urea, 1-(3-bromo-5-trifluoromethoxyphenyl)-3-(3-fluoro-2-hydroxymethylphenyl)urea, 1-(3-bromo-5-trifluoromethoxyphenyl)-3-(3-chloro-2-hydroxymethylphenyl)urea, 1-(3-bromo-2-hydroxymethylphenyl)-3-(3-bromo-5-trifluoromethoxyphenyl)urea, 1-(3-bromo-5-trifluoromethoxyphenyl)-3-(5-fluoro-2-hydroxymethylphenyl)urea, 1-(3-bromo-5-trifluoromethoxyphenyl)-3-(5-chloro-2-hydroxymethylphenyl)urea, 1-(5-bromo-2-hydroxymethylphenyl)-3-(3-bromo-5-trifluoromethoxyphenyl)urea, 1-(3-bromo-5-trifluoromethoxyphenyl)-3-(3,5-difluoro-2-hydroxymethylphenyl)urea, 1-(3-bromo-5-trifluoromethoxyphenyl)-3-(3,5-dichloro-2-hydroxymethylphenyl)urea, 1-(3,5-dibromo-2-hydroxymethylphenyl)-3-(3-bromo-5-trifluoromethoxyphenyl)urea,
**R1 is OCF₃, R2 is NHetOH:** 1-[3-(2-hydroxyethylamino)-5-trifluoromethoxyphenyl]-3-(2-hydroxymethylphenyl)urea, 1-(3-fluoro-2-hydroxymethylphenyl)-3-[3-(2-hydroxyethylamino)-5-trifluoromethoxyphenyl]urea, 1-(3-chloro-2-hydroxymethylphenyl)-3-[3-(2-hydroxyethylamino)-5-trifluoromethoxyphenyl]urea, 1-(3-bromo-2-hydroxymethylphenyl)-3-[3-(2-hydroxyethylamino)-5-trifluoromethoxyphenyl]urea, 1-(5-fluoro-2-hydroxymethylphenyl)-3-[3-(2-hydroxyethylamino)-5-trifluoromethoxyphenyl]urea, 1-(5-chloro-2-hydroxymethylphenyl)-3-[3-(2-hydroxyethylamino)-5-trifluoromethoxyphenyl]urea, 1-(5-bromo-2-hydroxymethylphenyl)-3-[3-(2-hydroxyethylamino)-5-trifluoromethoxyphenyl]urea, 1-(3,5-difluoro-2-hydroxymethylphenyl)-3-[3-(2-hydroxyethylamino)-5-trifluoromethoxyphenyl]urea, 1-(3,5-dichloro-2-hydroxymethylphenyl)-3-[3-(2-hydroxyethylamino)-5-trifluoromethoxyphenyl]urea, 1-(3, 5-dibromo-2-hydroxymethylphenyl)-3 -[3 -(2-hydroxyethylamino)-5-trifluoromethoxyphenyl]urea,
**R1 is OCF₃, R2 is NHetNH₂:** 1-[3-(2-aminoethylamino)-5-trifluoromethoxyphenyl]-3-(2-hydroxymethylphenyl)urea, 1-(3-fluoro-2-hydroxymethylphenyl)-3-[3-(2-aminoethylamino)-5-trifluoromethoxyphenyl]urea, 1-(3-chloro-2-hydroxymethylphenyl)-3-[3-(2-aminoethylamino)-5-trifluoromethoxyphenyl]urea, 1-(3-bromo-2-hydroxymethylphenyl)-3-[3-(2-aminoethylamino)-5-trifluoromethoxyphenyl]urea, 1-(5-fluoro-2-hydroxymethylphenyl)-3-[3-(2-aminoethylamino)-5-trifluoromethoxyphenyl]urea, 1-(5-chloro-2-hydroxymethylphenyl)-3-[3-(2-aminoethylamino)-5-trifluoromethoxyphenyl]urea, 1-(5-bromo-2-hydroxymethylphenyl)-3-[3-(2-aminoethylamino)-5-trifluoromethoxyphenyl]urea, 1-(3,5-difluoro-2-hydroxymethylphenyl)-3-[3-(2-aminoethylamino)-5-trifluoromethoxyphenyl]urea, 1-(3,5-dichloro-2-hydroxymethylphenyl)-3-[3-(2-aminoethylamino)-5-trifluoromethoxyphenyl]urea, 1-(3,5-dibromo-2-hydroxymethylphenyl)-3-[3-(2-aminoethylamino)-5-trifluoromethoxyphenyl]urea,
**R1 is OCH₃, R2 is H:** 1-(3-fluoro-2-hydroxymethylphenyl)-3-(3-methoxyphenyl)urea, 1-(3-chloro-2-hydroxymethylphenyl)-3-(3-methoxyphenyl)urea, 1-(3-bromo-2-hydroxymethylphenyl)-3-(3-methoxyphenyl)urea, 1-(5-fluoro-2-hydroxymethylphenyl)-3-(3-methoxyphenyl)urea, 1-(5-chloro-2-hydroxymethylphenyl)-3-(3-methoxyphenyl)urea, 1-(5-bromo-2-hydroxymethylphenyl)-3-(3-methoxyphenyl)urea, 1-(3,5-difluoro-2-hydroxymethylphenyl)-3-(3-methoxyphenyl)urea, 1-(3,5-dichloro-2-hydroxymethylphenyl)-3-(3-methoxyphenyl)urea, 1-(3,5-dibromo-2-hydroxymethylphenyl)-3-(3-methoxyphenyl)urea,
**R1 is OCH₃, R2 is F:** 1-(3-fluoro-5-methoxyphenyl)-3-(2-hydroxymethylphenyl)urea, 1-(3-fluoro-2-hydroxymethylphenyl)-3-(3-fluoro-5-methoxyphenyl)urea, 1-(3-chloro-2-hydroxymethylphenyl)-3-(3-fluoro-5-methoxyphenyl)urea, 1-(3-bromo-2-hydroxymethylphenyl)-3-(3-fluoro-5-methoxyphenyl)urea, 1-(5-fluoro-2-hydroxymethylphenyl)-3 -(3 -fluoro-5 -methoxyphenyl)urea, 1-(5-chloro-2-hydroxymethylphenyl)-3-(3-fluoro-5-methoxyphenyl)urea, 1-(5-bromo-2-hydroxymethylphenyl)-3-(3-fluoro-5-methoxyphenyl)urea, 1-(3,5-difluoro-2-hydroxymethylphenyl)-3-(3-fluoro-5-methoxyphenyl)urea, 1-(3,5-dichloro-2-hydroxymethylphenyl)-3-(3-fluoro-5-methoxyphenyl)urea, 1-(3, 5-dibromo-2-hydroxymethylphenyl)-3-(3-fluoro-5-methoxyphenyl)urea,
**R1 is OCH₃, R2 is Cl:** 1-(3-chloro-5-methoxyphenyl)-3-(2-hydroxymethylphenyl)urea, 1-(3-chloro-5-methoxyphenyl)-3-(3-fluoro-2-hydroxymethylphenyl)urea, 1-(3-chloro-2-hydroxymethylphenyl)-3-(3-chloro-5-methoxyphenyl)urea, 1-(3-bromo-2-hydroxymethylphenyl)-3-(3-chloro-5-methoxyphenyl)urea, 1-(5-fluoro-2-hydroxymethylphenyl)-3-(3-chloro-5-methoxyphenyl)urea, 1-(5-chloro-2-hydroxymethylphenyl)-3-(3-chloro-5-methoxyphenyl)urea, 1-(5-bromo-2-hydroxymethylphenyl)-3-(3-chloro-5-methoxyphenyl)urea, 1-(3,5-difluoro-2-hydroxymethylphenyl)-3-(3-chloro-5-methoxyphenyl)urea, 1-(3,5-dichloro-2-hydroxymethylphenyl)-3-(3-chloro-5-methoxyphenyl)urea, 1-(3,5-dibromo-2-hydroxymethylphenyl)-3-(3-chloro-5-methoxyphenyl)urea,
**R1 is OCH₃, R2 is Br:** 1-(3-bromo-5-methoxyphenyl)-3-(2-hydroxymethylphenyl)urea, 1-(3-bromo-5-methoxyphenyl)-3-(3-fluoro-2-hydroxymethylphenyl)urea, 1-(3-bromo-5-methoxyphenyl)-3-(3-chloro-2-hydroxymethylphenyl)urea, 1-(3-bromo-2-hydroxymethylphenyl)-3-(3-bromo-5-methoxyphenyl)urea, 1-(3-bromo-5-methoxyphenyl)-3-(5-fluoro-2-hydroxymethylphenyl)urea, 1-(3-bromo-5-methoxyphenyl)-3-(5-chloro-2-hydroxymethylphenyl)urea, 1-(5-bromo-2-hydroxymethylphenyl)-3-(3-bromo-5-methoxyphenyl)urea, 1-(3-bromo-5-methoxyphenyl)-3-(3,5-difluoro-2-hydroxymethylphenyl)urea, 1-(3-bromo-5-methoxyphenyl)-3-(3,5-dichloro-2-hydroxymethylphenyl)urea, 1-(3,5-dibromo-2-hydroxymethylphenyl)-3-(3-bromo-5-methoxyphenyl)urea,
**R1 is OCH₃, R2 is NHetOH:** 1-[3-(2-hydroxyethylamino)-5-methoxyphenyl]-3-(2-hydroxymethylphenyl)urea, 1-(3-fluoro-2-hydroxymethylphenyl)-3-[3-(2-hydroxyethylamino)-5-methoxyphenyl]urea, 1-(3-chloro-2-hydroxymethylphenyl)-3-[3-(2-hydroxyethylamino)-5-methoxyphenyl]urea, 1-(3-bromo-2-hydroxymethylphenyl)-3-[3-(2-hydroxyethylamino)-5-methoxyphenyl]urea, 1-(5-fluoro-2-hydroxymethylphenyl)-3-[3-(2-hydroxyethylamino)-5-methoxyphenyl]urea, 1-(5-chloro-2-hydroxymethylphenyl)-3-[3-(2-hydroxyethylamino)-5-methoxyphenyl]urea, 1-(5-bromo-2-hydroxymethylphenyl)-3-[3-(2-hydroxyethylamino)-5-methoxyphenyl]urea, 1-(3,5-difluoro-2-hydroxymethylphenyl)-3-[3-(2-hydroxyethylamino)-5-methoxyphenyl]urea, 1-(3,5-dichloro-2-hydroxymethylphenyl)-3-[3-(2-hydroxyethylamino)-5-methoxyphenyl]urea, 1-(3,5-dibromo-2-hydroxymethylphenyl)-3-[3-(2-hydroxyethylamino)-5-methoxyphenyl]urea,
**R1 is OCH₃, R2 is NHetNH₂:** 1-[3-(2-aminoethylamino)-5-methoxyphenyl]-3-(2-hydroxymethylphenyl)urea, 1-(3-fluoro-2-hydroxymethylphenyl)-3-[3-(2-aminoethylamino)-5-methoxyphenyl]urea, 1-(3-chloro-2-hydroxymethylphenyl)-3-[3-(2-aminoethylamino)-5-methoxyphenyl]urea, 1-(3-bromo-2-hydroxymethylphenyl)-3-[3-(2-aminoethylamino)-5-methoxyphenyl]urea, 1-(5-fluoro-2-hydroxymethylphenyl)-3-[3-(2-aminoethylamino)-5-methoxyphenyl]urea, 1-(5-chloro-2-hydroxymethylphenyl)-3-[3-(2-aminoethylamino)-5-methoxyphenyl]urea, 1-(5-bromo-2-hydroxymethylphenyl)-3-[3-(2-aminoethylamino)-5-methoxyphenyl]urea, 1-(3,5-difluoro-2-hydroxymethylphenyl)-3-[3-(2-aminoethylamino)-5-methoxyphenyl]urea, 1-(3,5-dichloro-2-hydroxymethylphenyl)-3-[3-(2-aminoethylamino)-5-methoxyphenyl]urea, 1-(3,5-dibromo-2-hydroxymethylphenyl)-3-[3-(2-aminoethylamino)-5-methoxyphenyl]urea,
**R1 is Br, R2 is H:** 1-(3-bromophenyl)-3-(2-hydroxymethylphenyl)urea, 1-(3-bromophenyl)-3-(3-fluoro-2-hydroxymethylphenyl)urea, 1-(3 -bromophenyl)-3 -(3 -chloro-2-hydroxymethylphenyl)urea, 1-(3 -bromo-2-hydroxymethylphenyl)-3 -(3 -bromophenyl)urea, 1-(3-bromophenyl)-3-(5-fluoro-2-hydroxymethylphenyl)urea, 1-(3-bromophenyl)-3-(5-chloro-2-hydroxymethylphenyl)urea, 1-(5 -bromo-2-hydroxymethylphenyl)-3 -(3 -bromophenyl)urea, 1-(3-bromophenyl)-3-(3,5-difluoro-2-hydroxymethylphenyl)urea, 1-(3-bromophenyl)-3-(3,5-dichloro-2-hydroxymethylphenyl)urea, 1-(3,5 -dibromo-2-hydroxymethylphenyl)-3 -(3 -bromophenyl)urea,
**R1 is Br, R2 is F:** 1-(3-bromo-5-fluorophenyl)-3-(2-hydroxymethylphenyl)urea, 1-(3-bromo-5-fluorophenyl)-3-(3-fluoro-2-hydroxymethylphenyl)urea, 1-(3-bromo-5-fluorophenyl)-3-(3-chloro-2-hydroxymethylphenyl)urea, 1-(3-bromo-5-fluorophenyl)-3-(3-bromo-2-hydroxymethylphenyl)urea, 1-(3-bromo-5-fluorophenyl)-3-(5-fluoro-2-hydroxymethylphenyl)urea, 1-(3-bromo-5-fluorophenyl)-3-(5-chloro-2-hydroxymethylphenyl)urea, 1-(3-bromo-5-fluorophenyl)-3-(5-bromo-2-hydroxymethylphenyl)urea, 1-(3 -bromo-5 -fluorophenyl)-3 -(3,5 -difluoro-2-hydroxymethylphenyl)urea, 1-(3-bromo-5-fluorophenyl)-3-(3,5-dichloro-2-hydroxymethylphenyl)urea, 1-(3-bromo-5-fluorophenyl)-3-(3,5-dibromo-2-hydroxymethylphenyl)urea,
**R1 is Br, R2 is Cl:** 1-(3-bromo-5-chlorophenyl)-3-(2-hydroxymethylphenyl)urea, 1-(3-bromo-5-chlorophenyl)-3-(3-fluoro-2-hydroxymethylphenyl)urea, 1-(3-bromo-5-chlorophenyl)-3-(3-chloro-2-hydroxymethylphenyl)urea, 1-(3-bromo-5-chlorophenyl)-3-(3-bromo-2-hydroxymethylphenyl)urea, 1-(3-bromo-5-chlorophenyl)-3-(5-fluoro-2-hydroxymethylphenyl)urea, 1-(3-bromo-5-chlorophenyl)-3-(5-chloro-2-hydroxymethylphenyl)urea, 1-(3-bromo-5-chlorophenyl)-3-(5-bromo-2-hydroxymethylphenyl)urea, 1-(3-bromo-5-chlorophenyl)-3-(3,5-difluoro-2-hydroxymethylphenyl)urea, 1-(3-bromo-5-chlorophenyl)-3-(3,5-dichloro-2-hydroxymethylphenyl)urea, 1-(3-bromo-5-chlorophenyl)-3-(3,5-dibromo-2-hydroxymethylphenyl)urea,
**R1 is Br, R2 is Br:** 1-(3,5-dibromophenyl)-3-(2-hydroxymethylphenyl)urea, 1-(3,5-dibromophenyl)-3-(3-fluoro-2-hydroxymethylphenyl)urea, 1-(3,5-dibromophenyl)-3-(3-chloro-2-hydroxymethylphenyl)urea, 1-(3,5-dibromophenyl)-3-(3-bromo-2-hydroxymethylphenyl)urea, 1-(3,5-dibromophenyl)-3-(5-fluoro-2-hydroxymethylphenyl)urea, 1-(3,5-dibromophenyl)-3-(5-chloro-2-hydroxymethylphenyl)urea, 1-(3,5-dibromophenyl)-3-(5-bromo-2-hydroxymethylphenyl)urea, 1-(3,5-dibromophenyl)-3-(3,5-difluoro-2-hydroxymethylphenyl)urea, 1-(3,5-dibromophenyl)-3-(3,5-dichloro-2-hydroxymethylphenyl)urea, 1-(3,5-dibromophenyl)-3-(3,5-dibromo-2-hydroxymethylphenyl)urea,
**R1 is Br, R2 is NHetOH:** 1-[3-bromo-5-(2-hydroxyethylamino)phenyl]-3-(2-hydroxymethylphenyl)urea, 1-[3-bromo-5-(2-hydroxyethylamino)phenyl]-3-(3-fluoro-2-hydroxymethylphenyl)urea, 1-[3-bromo-5-(2-hydroxyethylamino)phenyl]-3-(3-chloro-2-hydroxymethylphenyl)urea, 1-[3-bromo-5-(2-hydroxyethylamino)phenyl]-3-(3-bromo-2-hydroxymethylphenyl)urea, 1-[3-bromo-5-(2-hydroxyethylamino)phenyl]-3-(5-fluoro-2-hydroxymethylphenyl)urea, 1-[3-bromo-5-(2-hydroxyethylamino)phenyl]-3-(5-chloro-2-hydroxymethylphenyl)urea, 1-[3-bromo-5-(2-hydroxyethylamino)phenyl]-3-(5-bromo-2-hydroxymethylphenyl)urea, 1-[3-bromo-5-(2-hydroxyethylamino)phenyl]-3-(3,5-difluoro-2-hydroxymethylphenyl)urea, 1-[3-bromo-5-(2-hydroxyethylamino)phenyl]-3-(3,5-dichloro-2-hydroxymethylphenyl)urea, 1-[3-bromo-5-(2-hydroxyethylamino)phenyl]-3-(3,5-dibromo-2-hydroxymethylphenyl)urea,
**R1 is Br, R2 is NHetNH₂:** 1-[3-bromo-5-(2-aminoethylamino)phenyl]-3-(2-hydroxymethylphenyl)urea, 1-[3-bromo-5-(2-aminoethylamino)phenyl]-3-(3-fluoro-2-hydroxymethylphenyl)urea, 1-[3-bromo-5-(2-aminoethylamino)phenyl]-3-(3-chloro-2-hydroxymethylphenyl)urea, 1-[3-bromo-5-(2-aminoethylamino)phenyl]-3-(3-bromo-2-hydroxymethylphenyl)urea, 1-[3-bromo-5-(2-aminoethylamino)phenyl]-3-(5-fluoro-2-hydroxymethylphenyl)urea, 1-[3-bromo-5-(2-aminoethylamino)phenyl]-3-(5-chloro-2-hydroxymethylphenyl)urea, 1-[3-bromo-5-(2-aminoethylamino)phenyl]-3-(5-bromo-2-hydroxymethylphenyl)urea, 1-[3-bromo-5-(2-aminoethylamino)phenyl]-3-(3,5-difluoro-2-hydroxymethylphenyl)urea, 1-[3-bromo-5-(2-aminoethylamino)phenyl]-3-(3,5-dichloro-2-hydroxymethylphenyl)urea, 1-[3-bromo-5-(2-aminoethylamino)phenyl]-3-(3,5-dibromo-2-hydroxymethylphenyl)urea,
**R1 is Cl, R2 is H:** 1-(3-chlorophenyl)-3-(3-fluoro-2-hydroxymethylphenyl)urea, 1-(3-chlorophenyl)-3-(3-chloro-2-hydroxymethylphenyl)urea, 1-(3-chlorophenyl)-3-(3-bromo-2-hydroxymethylphenyl)urea, 1-(3-chlorophenyl)-3-(5-fluoro-2-hydroxymethylphenyl)urea, 1-(3-chlorophenyl)-3-(5-chloro-2-hydroxymethylphenyl)urea, 1-(3-chlorophenyl)-3-(5-bromo-2-hydroxymethylphenyl)urea, 1-(3-chlorophenyl)-3-(3,5-difluoro-2-hydroxymethylphenyl)urea, 1-(3 -chlorophenyl)-3 -(3,5 -dichloro-2-hydroxymethylphenyl)urea, 1-(3 -chlorophenyl)-3 -(3,5-dibromo-2-hydroxymethylphenyl)urea,
**R1 is Cl, R2 is F:** 1-(3-chloro-5-fluorophenyl)-3-(2-hydroxymethylphenyl)urea, 1-(3-chloro-5-fluorophenyl)-3-(3-fluoro-2-hydroxymethylphenyl)urea, 1-(3-chloro-5-fluorophenyl)-3-(3-chloro-2-hydroxymethylphenyl)urea, 1-(3-chloro-5-fluorophenyl)-3-(3-bromo-2-hydroxymethylphenyl)urea, 1-(3-chloro-5-fluorophenyl)-3-(5-fluoro-2-hydroxymethylphenyl)urea, 1-(3-chloro-5-fluorophenyl)-3-(5-chloro-2-hydroxymethylphenyl)urea, 1-(3-chloro-5-fluorophenyl)-3-(5-bromo-2-hydroxymethylphenyl)urea, 1-(3-chloro-5-fluorophenyl)-3-(3,5-difluoro-2-hydroxymethylphenyl)urea, 1-(3-chloro-5-fluorophenyl)-3-(3,5-dichloro-2-hydroxymethylphenyl)urea, 1-(3-chloro-5-fluorophenyl)-3-(3,5-dibromo-2-hydroxymethylphenyl)urea,
**R1 is Cl, R2 is Cl:** 1-(3,5-dichlorophenyl)-3-(3-fluoro-2-hydroxymethylphenyl)urea, 1-(3,5-dichlorophenyl)-3-(3-chloro-2-hydroxymethylphenyl)urea, 1-(3,5-dichlorophenyl)-3-(3-bromo-2-hydroxymethylphenyl)urea, 1-(3,5-dichlorophenyl)-3-(5-fluoro-2-hydroxymethylphenyl)urea, 1-(3,5-dichlorophenyl)-3-(5-chloro-2-hydroxymethylphenyl)urea, 1-(3,5-dichlorophenyl)-3-(5-bromo-2-hydroxymethylphenyl)urea, 1-(3,5-dichlorophenyl)-3-(3,5-difluoro-2-hydroxymethylphenyl)urea, 1-(3,5-dichlorophenyl)-3-(3,5-dichloro-2-hydroxymethylphenyl)urea, 1-(3,5-dichlorophenyl)-3-(3,5-dibromo-2-hydroxymethylphenyl)urea,
**R1 is Cl, R2 is NHetOH:** 1-[3-chloro-5-(2-hydroxyethylamino)phenyl]-3-(2-hydroxymethylphenyl)urea, 1-[3-chloro-5-(2-hydroxyethylamino)phenyl]-3-(3-fluoro-2-hydroxymethylphenyl)urea, 1-[3-chloro-5-(2-hydroxyethylamino)phenyl]-3-(3-chloro-2-hydroxymethylphenyl)urea, 1-[3-chloro-5-(2-hydroxyethylamino)phenyl]-3-(3-bromo-2-hydroxymethylphenyl)urea, 1-[3-chloro-5-(2-hydroxyethylamino)phenyl]-3-(5-fluoro-2-hydroxymethylphenyl)urea, 1-[3-chloro-5-(2-hydroxyethylamino)phenyl]-3-(5-chloro-2-hydroxymethylphenyl)urea, 1-[3-chloro-5-(2-hydroxyethylamino)phenyl]-3-(5-bromo-2-hydroxymethylphenyl)urea, 1-[3-chloro-5-(2-hydroxyethylamino)phenyl]-3-(3,5-difluoro-2-hydroxymethylphenyl)urea, 1-[3-chloro-5-(2-hydroxyethylamino)phenyl]-3-(3,5-dichloro-2-hydroxymethylphenyl)urea, 1-[3-chloro-5-(2-hydroxyethylamino)phenyl]-3-(3,5-dibromo-2-hydroxymethylphenyl)urea,
**R1 is Cl, R2 is NHetNH₂:** 1-[3-chloro-5-(2-aminoethylamino)phenyl]-3-(2-hydroxymethylphenyl)urea, 1-[3-chloro-5-(2-aminoethylamino)phenyl]-3-(3-fluoro-2-hydroxymethylphenyl)urea, 1-[3-chloro-5-(2-aminoethylamino)phenyl]-3-(3-chloro-2-hydroxymethylphenyl)urea, 1-[3-chloro-5-(2-aminoethylamino)phenyl]-3-(3-bromo-2-hydroxymethylphenyl)urea, 1-[3-chloro-5-(2-aminoethylamino)phenyl]-3-(5-fluoro-2-hydroxymethylphenyl)urea, 1-[3-chloro-5-(2-aminoethylamino)phenyl]-3-(5-chloro-2-hydroxymethylphenyl)urea, 1-[3-chloro-5-(2-aminoethylamino)phenyl]-3-(5-bromo-2-hydroxymethylphenyl)urea, 1-[3-chloro-5-(2-aminoethylamino)phenyl]-3-(3,5-difluoro-2-hydroxymethylphenyl)urea, 1-[3-chloro-5-(2-aminoethylamino)phenyl]-3-(3,5-dichloro-2-hydroxymethylphenyl)urea, 1-[3-chloro-5-(2-aminoethylamino)phenyl]-3-(3,5-dibromo-2-hydroxymethylphenyl)urea,
**R1 is F, R2 is H:** 1-(3-fluorophenyl) -3-(2-hydroxymethylphenyl)urea, 1-(3-fluoro-2-hydroxymethylphenyl)-3-(3-fluorophenyl)urea, 1-(3-chloro-2-hydroxymethylphenyl)-3-(3-fluorophenyl)urea, 1-(3-bromo-2-hydroxymethylphenyl)-3-(3-fluorophenyl)urea, 1-(5 -fluoro-2-hydroxymethylphenyl)-3 -(3 -fluorophenyl)urea, 1-(5-chloro-2-hydroxymethylphenyl)-3-(3-fluorophenyl)urea, 1-(5-bromo-2-hydroxymethylphenyl)-3-(3-fluorophenyl)urea, 1-(3,5-difluoro-2-hydroxymethylphenyl)-3-(3-fluorophenyl)urea, 1-(3,5-dichloro-2-hydroxymethylphenyl) -3-(3-fluorophenyl)urea, 1-(3,5-dibromo-2-hydroxymethylphenyl)-3-(3-fluorophenyl)urea,
**R1 is F, R2 is F:** 1-(3,5-difluorophenyl)-3-(2-hydroxymethylphenyl)urea, 1-(3-fluoro-2-hydroxymethylphenyl)-3-(3,5-difluorophenyl)urea, 1-(3-chloro-2-hydroxymethylphenyl)-3-(3,5-difluorophenyl)urea, 1-(3 -bromo-2-hydroxymethylphenyl)-3 -(3,5 -difluorophenyl)urea, 1-(5-fluoro-2-hydroxymethylphenyl)-3-(3,5-difluorophenyl)urea, 1-(5-chloro-2-hydroxymethylphenyl)-3-(3,5-difluorophenyl)urea, 1-(5-bromo-2-hydroxymethylphenyl)-3-(3,5-difluorophenyl)urea, 1-(3,5-difluoro-2-hydroxymethylphenyl)-3-(3,5-difluorophenyl)urea, 1-(3,5-dichloro-2-hydroxymethylphenyl)-3-(3,5-difluorophenyl)urea, 1-(3,5-dibromo-2-hydroxymethylphenyl)-3-(3,5-difluorophenyl)urea,
**R1 is F, R2 is NHetOH:** 1-[3-fluoro-5-(2-hydroxyethylamino)phenyl]-3-(2-hydroxymethylphenyl)urea, 1-[3-fluoro-5-(2-hydroxyethylamino)phenyl]-3-(3-fluoro-2-hydroxymethylphenyl)urea, 1-[3-fluoro-5-(2-hydroxyethylamino)phenyl]-3-(3-chloro-2-hydroxymethylphenyl)urea, 1-[3-fluoro-5-(2-hydroxyethylamino)phenyl]-3-(3-bromo-2-hydroxymethylphenyl)urea, 1-[3-fluoro-5-(2-hydroxyethylamino)phenyl]-3-(5-fluoro-2-hydroxymethylphenyl)urea, 1-[3-fluoro-5-(2-hydroxyethylamino)phenyl]-3-(5-chloro-2-hydroxymethylphenyl)urea, 1-[3-fluoro-5-(2-hydroxyethylamino)phenyl]-3-(5-bromo-2-hydroxymethylphenyl)urea, 1-[3-fluoro-5-(2-hydroxyethylamino)phenyl]-3-(3,5-difluoro-2-hydroxymethylphenyl)urea, 1-[3-fluoro-5-(2-hydroxyethylamino)phenyl]-3-(3,5-dichloro-2-hydroxymethylphenyl)urea, 1-[3-fluoro-5-(2-hydroxyethylamino)phenyl]-3-(3,5-dibromo-2-hydroxymethylphenyl)urea,
**R1 is F, R2 is NHetNH₂:** 1-[3-fluoro-5-(2-aminoethylamino)phenyl]-3-(2-hydroxymethylphenyl)urea, 1-[3-fluoro-5-(2-aminoethylamino)phenyl]-3-(3-fluoro-2-hydroxymethylphenyl)urea, 1-[3-fluoro-5-(2-aminoethylamino)phenyl]-3-(3-chloro-2-hydroxymethylphenyl)urea, 1-[3-fluoro-5-(2-aminoethylamino)phenyl]-3-(3-bromo-2-hydroxymethylphenyl)urea, 1-[3-fluoro-5-(2-aminoethylamino)phenyl]-3-(5-fluoro-2-hydroxymethylphenyl)urea, 1-[3-fluoro-5-(2-aminoethylamino)phenyl]-3-(5-chloro-2-hydroxymethylphenyl)urea, 1-[3-fluoro-5-(2-aminoethylamino)phenyl]-3-(5-bromo-2-hydroxymethylphenyl)urea, 1-[3-fluoro-5-(2-aminoethylamino)phenyl]-3-(3,5-difluoro-2-hydroxymethylphenyl)urea, 1-[3-fluoro-5-(2-aminoethylamino)phenyl]-3-(3,5-dichloro-2-hydroxymethylphenyl)urea, 1-[3-fluoro-5-(2-aminoethylamino)phenyl]-3-(3,5-dibromo-2-hydroxymethylphenyl)urea.

In one preferred embodiment, X = N, n = 2 and the compound of the general formula (I) is selected from the group comprising:
**R1 is F, R2 is H:** 1-(2-fluoropyridin-4-yl)-3-[2-(2-hydroxyethyl)phenyl]urea, 1-[3-chloro-2-(2-hydroxyethyl)phenyl]-3-(2-fluoropyridin-4-yl)urea, 1-[5-chloro-2-(2-hydroxyethyl)phenyl]-3-(2-fluoropyridin-4-yl)urea, 1-[5-fluoro-2-(2-hydroxyethyl)phenyl]-3-(2-fluoropyridin-4-yl)urea, 1-[3,5-dichloro-2-(2-hydroxyethyl)phenyl]-3-(2-fluoropyridin-4-yl)urea,
**R1 is F, R2 is F:** 1-(2,6-difluoropyridin-4-yl)-3-[2-(2-hydroxyethyl)phenyl]urea, 1-[3-chloro-2-(2-hydroxyethyl)phenyl]-3-(2,6-difluoropyridin-4-yl)urea, 1-[5-chloro-2-(2-hydroxyethyl)phenyl]-3-(2,6-difluoropyridin-4-yl)urea, 1-[5-fluoro-2-(2-hydroxyethyl)phenyl]-3-(2,6-difluoropyridin-4-yl)urea, 1-[3,5-dichloro-2-(2-hydroxyethyl)phenyl]-3-(2,6-difluoropyridin-4-yl)urea,
**R1 is Cl, R2 is H:** 1-(2-chloropyridin-4-yl)-3-[2-(2-hydroxyethyl)phenyl]urea, 1-[3-chloro-2-(2-hydroxyethyl)phenyl]-3-(2-chloropyridin-4-yl)urea, 1-[5-chloro-2-(2-hydroxyethyl)phenyl]-3-(2-chloropyridin-4-yl)urea, 1-(2-chloropyridin-4-yl)-3-[5-fluoro-2-(2-hydroxyethyl)phenyl]urea, 1-[3,5-dichloro-2-(2-hydroxyethyl)phenyl]-3-(2-chloropyridin-4-yl)urea,
**R1 is Cl, R2 is Cl:** 1-[3-chloro-2-(2-hydroxyethyl)phenyl]-3-(2,6-dichloropyridin-4-yl)urea, 1-[5-chloro-2-(2-hydroxyethyl)phenyl]-3-(2,6-dichloropyridin-4-yl)urea, 1-[5-fluoro-2-(2-hydroxyethyl)phenyl]-3-(2,6-dichloropyridin-4-yl)urea, 1-[3,5-dichloro-2-(2-hydroxyethyl)phenyl]-3-(2,6-dichloropyridin-4-yl)urea,
**R1 is Br, R2 is H:** 1-(2-bromopyridin-4-yl)-3-[2-(2-hydroxyethyl)phenyl]urea, 1-(2-bromopyridin-4-yl)-3-[3-chloro-2-(2-hydroxyethyl)phenyl]urea, 1-(2-bromopyridin-4-yl)-3-[5-chloro-2-(2-hydroxyethyl)phenyl]urea, 1-(2-bromopyridin-4-yl)-3-[5-fluoro-2-(2-hydroxyethyl)phenyl]urea, 1-(2-bromopyridin-4-yl)-3-[3,5-dichloro-2-(2-hydroxyethyl)phenyl]urea,
**R1 is OCH₃, R2 is H:** 1-[2-(2-hydroxyethyl)phenyl]-3-(2-methoxypyridin-4-yl)urea, 1-[3-chloro-2-(2-hydroxyethyl)phenyl]-3-(2-methoxypyridin-4-yl)urea, 1-[5-chloro-2-(2-hydroxyethyl)phenyl]-3-(2-methoxypyridin-4-yl)urea, 1-[5-fluoro-2-(2-hydroxyethyl)phenyl]-3-(2-methoxypyridin-4-yl)urea, 1-[3,5-dichloro-2-(2-hydroxyethyl)phenyl]-3-(2-methoxypyridin-4-yl)urea.

In one preferred embodiment, X = C, n = 2 and the compound of the general formula (I) is selected from the group comprising:
**R1 is SCF₃, R2 is H:** 1-[2-(2-hydroxyethyl)phenyl]-3-(3-trifluoromethylsulphanylphenyl)urea, 1-[3-chloro-2-(2-hydroxyethyl)phenyl]-3-(3-trifluoromethylsulphanylphenyl)urea, 1-[5-chloro-2-(2-hydroxyethyl)phenyl]-3-(3-trifluoromethylsulphanylphenyl)urea, 1-[5-fluoro-2-(2-hydroxyethyl)phenyl]-3-(3-trifluoromethylsulphanylphenyl)urea, 1-[3,5-dichloro-2-(2-hydroxyethyl)phenyl]-3-(3-trifluoromethylsulphanylphenyl)urea,
**R1 is SCH₃, R2 is H:** 1-[2-(2-hydroxyethyl)phenyl]-3-(3-methylsulphanylphenyl)urea, 1-[3-chloro-2-(2-hydroxyethyl)phenyl]-3-(3-methylsulphanylphenyl)urea, 1-[5-chloro-2-(2-hydroxyethyl)phenyl]-3-(3-methylsulphanylphenyl)urea, 1-[5-fluoro-2-(2-hydroxyethyl)phenyl]-3-(3-methylsulphanylphenyl)urea, 1-[3,5-dichloro-2-(2-hydroxyethyl)phenyl]-3-(3-methylsulphanylphenyl)urea,
R**1 is OCF₃, R2 is H:** 1-[3-chloro-2-(2-hydroxyethyl)phenyl]-3-(3-trifluoromethoxyphenyl)urea, 1-[5-chloro-2-(2-hydroxyethyl)phenyl]-3-(3-trifluoromethoxyphenyl)urea, 1-[5-fluoro-2-(2-hydroxyethyl)phenyl]-3-(3-trifluoromethoxyphenyl)urea, 1-[3,5-dichloro-2-(2-hydroxyethyl)phenyl]-3-(3-trifluoromethoxyphenyl)urea,
**R1 is OCF₃, R2 is F:** 1-(3-fluoro-5-trifluoromethoxyphenyl)-3-[2-(2-hydroxyethyl)phenyl]urea, 1-[3-chloro-2-(2-hydroxyethyl)phenyl]-3-(3-fluoro-5-trifluoromethoxyphenyl)urea, 1-[5-chloro-2-(2-hydroxyethyl)phenyl]-3-(3-fluoro-5-trifluoromethoxyphenyl)urea, 1-[5-fluoro-2-(2-hydroxyethyl)phenyl]-3-(3-fluoro-5-trifluoromethoxyphenyl)urea, 1-[3,5-dichloro-2-(2-hydroxyethyl)phenyl]-3-(3-fluoro-5-trifluoromethoxyphenyl)urea,
**R1 is OCF₃, R2 is Cl:** 1-(3-chloro-5-trifluoromethoxyphenyl)-3-[2-(2-hydroxyethyl)phenyl]urea, 1-[3-chloro-2-(2-hydroxyethyl)phenyl]-3-(3-chloro-5-trifluoromethoxyphenyl)urea, 1-[5-chloro-2-(2-hydroxyethyl)phenyl]-3-(3-chloro-5-trifluoromethoxyphenyl)urea, 1-(3-chloro-5-trifluoromethoxyphenyl)-3-[5-fluoro-2-(2-hydroxyethyl)phenyl]urea, 1-[3,5-dichloro-2-(2-hydroxyethyl)phenyl]-3-(3-chloro-5-trifluoromethoxyphenyl)urea,
**R1 is OCF₃, R2 is Br:** 1-(3-bromo-5-trifluoromethoxyphenyl)-3-[2-(2-hydroxyethyl)phenyl]urea, 1-(3-bromo-5-trifluoromethoxyphenyl)-3-[3-chloro-2-(2-hydroxyethyl)phenyl]urea, 1-(3-bromo-5-trifluoromethoxyphenyl)-3-[5-chloro-2-(2-hydroxyethyl)phenyl]urea, 1-(3-bromo-5-trifluoromethoxyphenyl)-3-[5-fluoro-2-(2-hydroxyethyl)phenyl]urea, 1-(3-bromo-5-trifluoromethoxyphenyl)-3-[3,5-dichloro-2-(2-hydroxyethyl)phenyl]urea,
**R1 is OCF₃, R2 is NHetOH:** 1-[3-(2-hydroxyethylamino)-5-trifluoromethoxyphenyl]-3-[2-(2-hydroxyethyl)phenyl]urea, 1-[3-chloro-2-(2-hydroxyethyl)phenyl]-3-[3-(2-hydroxyethylamino)-5-trifluoromethoxyphenyl]urea, 1-[5-chloro-2-(2-hydroxyethyl)phenyl]-3-[3-(2-hydroxyethylamino)-5-trifluoromethoxyphenyl]urea, 1-[5-fluoro-2-(2-hydroxyethyl)phenyl]-3-[3-(2-hydroxyethylamino)-5-trifluoromethoxyphenyl]urea, 1-[3,5-dichloro-2-(2-hydroxyethyl)phenyl]-3-[3-(2-hydroxyethylamino)-5-trifluoromethoxyphenyl]urea,
**R1 is OCF₃, R2 is NHetNH₂:** 1-[3-(2-aminoethylamino)-5-trifluoromethoxyphenyl]-3-[2-(2-hydroxyethyl)phenylurea, 1-[3-chloro-2-(2-hydroxyethyl)phenyl]-3-[3-(2-aminoethylamino)-5-trifluoromethoxyphenyl]urea, 1-[5-chloro-2-(2-hydroxyethyl)phenyl]-3-[3-(2-aminoethylamino)-5-trifluoromethoxyphenyl]urea, 1-[5-fluoro-2-(2-hydroxyethyl)phenyl]-3-[3-(2-aminoethylamino)-5-trifluoromethoxyphenyl]urea, 1-[3,5-dichloro-2-(2-hydroxyethyl)phenyl]-3-[3-(2-aminoethylamino)-5-trifluoromethoxyphenyl]urea,
**R1 is OCH₃, R2 is H:** 1-[3-chloro-2-(2-hydroxyethyl)phenyl]-3-(3-methoxyphenyl)urea, 1-[5-chloro-2-(2-hydroxyethyl)phenyl]-3-(3-methoxyphenyl)urea, 1-[5-fluoro-2-(2-hydroxyethyl)phenyl]-3-(3-methoxyphenyl)urea, 1-[3,5-dichloro-2-(2-hydroxyethyl)phenyl]-3-(3 -methoxyphenyl)urea,
**R1 is OCH₃, R2 is F:** 1-(3-fluoro-5-methoxyphenyl)-3-[2-(2-hydroxyethyl)phenyl]urea, 1-[3-chloro-2-(2-hydroxyethyl)phenyl]-3-(3-fluoro-5-methoxyphenyl)urea, 1-[5-chloro-2-(2-hydroxyethyl)phenyl]-3-(3-fluoro-5-methoxyphenyl)urea, 1-[5-fluoro-2-(2-hydroxyethyl)phenyl]-3-(3-fluoro-5-methoxyphenyl)urea, 1-[3, 5-dichloro-2-(2-hydroxyethyl)phenyl]-3-(3-fluoro-5-methoxyphenyl)urea,
**R1 is OCH₃, R2 is Cl:** 1-(3-chloro-5-methoxyphenyl)-3-[2-(2-hydroxyethyl)phenyl]urea, 1-[3-chloro-2-(2-hydroxyethyl)phenyl]-3-(3-chloro-5-methoxyphenyl)urea, 1-[5-chloro-2-(2-hydroxyethyl)phenyl]-3-(3-chloro-5-methoxyphenyl)urea, 1-(3-chloro-5-methoxyphenyl)-3-[5-fluoro-2-(2-hydroxyethyl)phenyl]urea, 1-[3,5-dichloro-2-(2-hydroxyethyl)phenyl]-3-(3-chloro-5-methoxyphenyl)urea,
**R1 is OCH₃, R2 is Br:** 1-(3-bromo-5-methoxyphenyl)-3-[2-(2-hydroxyethyl)phenyl]urea, 1-(3-bromo-5-methoxyphenyl)-3-[3-chloro-2-(2-hydroxyethyl)phenyl]urea, 1-(3-bromo-5-methoxyphenyl)-3-[5-chloro-2-(2-hydroxyethyl)phenyl]urea, 1-(3-bromo-5-methoxyphenyl)-3-[5-fluoro-2-(2-hydroxyethyl)phenyl]urea, 1-(3-bromo-5-methoxyphenyl)-3-[3,5-dichloro-2-(2-hydroxyethyl)phenyl]urea,
**R1 is OCH₃, R2 is NHetOH:** 1-[3-(2-hydroxyethylamino)-5-methoxyphenyl]-3- [2-(2-hydroxyethyl)phenyl]urea,1-[3-chloro-2-(2-hydroxyethyl)phenyl]-3-[3-(2-hydroxyethylamino)-5-methoxyphenyl]urea, 1-[5-chloro-2-(2-hydroxyethyl)phenyl]-3-[3-(2-hydroxyethylamino)-5-methoxyphenyl]urea, 1-[5-fluoro-2-(2-hydroxyethyl)phenyl]-3-[3-(2-hydroxyethylamino)-5-methoxyphenyl]urea, 1-[3,5-dichloro-2-(2-hydroxyethyl)phenyl]-3-[3-(2-hydroxyethylamino)-5-methoxyphenyl]urea,
**R1 is OCH₃, R2 is NHetNH₂:** 1-[3-(2-aminoethylamino)-5-methoxyphenyl]-3-[2-(2-hydroxyethyl)phenylurea, 1-[3-chloro-2-(2-hydroxyethyl)phenyl]-3-[3-(2-aminoethylamino)-5-methoxyphenyl]urea, 1-[5-chloro-2-(2-hydroxyethyl)phenyl]-3-[3-(2-aminoethylamino)-5-methoxyphenyl]urea, 1-[5-fluoro-2-(2-hydroxyethyl)phenyl]-3-[3-(2-aminoethylamino)-5-methoxyphenyl]urea, 1-[3,5-dichloro-2-(2-hydroxyethyl)phenyl]-3-[3-(2-aminoethylamino)-5-methoxyphenyl]urea,
**R1 is Br, R2 is H:** 1-(3-bromophenyl)-3-[2-(2-hydroxyethyl)phenyl]urea, 1-(3-bromophenyl)-3-[3-chloro-2-(2-hydroxyethyl)phenyl]urea, 1-(3-bromophenyl)-3-[5-chloro-2-(2-hydroxyethyl)phenyl]urea, 1-(3-bromophenyl)-3-[5-fluoro-2-(2-hydroxyethyl)phenyl]urea, 1-(3-bromophenyl)-3-[3,5-dichloro-2-(2-hydroxyethyl)phenyl]urea,
**R1 is Br, R2 is F:** 1-(3-bromo-5-fluorophenyl)-3-[2-(2-hydroxyethyl)phenyl]urea, 1-(3-bromo-5-fluorophenyl)-3-[3-chloro-2-(2-hydroxyethyl)phenyl]urea, 1-(3-bromo-5-fluorophenyl)-3-[5-chloro-2-(2-hydroxyethyl)phenyl]urea, 1-(3-bromo-5-fluorophenyl)-3-[5-fluoro-2-(2-hydroxyethyl)phenyl]urea, 1-(3-bromo-5-fluorophenyl)-3-[3,5-dichloro-2-(2-hydroxyethyl)phenyl]urea,
**R1 is Br, R2 is Cl:** 1-(3-bromo-5-chlorophenyl)-3-[2-(2-hydroxyethyl)phenyl]urea, 1-(3-bromo-5-chlorophenyl)-3-[3-chloro-2-(2-hydroxyethyl)phenyl]urea, 1-(3-bromo-5-chlorophenyl)-3-[5-chloro-2-(2-hydroxyethyl)phenyl]urea, 1-(3-bromo-5-chlorophenyl)-3-[5-fluoro-2-(2-hydroxyethyl)phenyl]urea, 1-(3-bromo-5-chlorophenyl)-3-[3,5-dichloro-2-(2-hydroxyethyl)phenyl]urea,
**R1 is Br, R2 is Br:** 1-(3,5-dibromophenyl)-3-[2-(2-hydroxyethyl)phenyl]urea, 1-(3,5-dibromophenyl)-3-[3-chloro-2-(2-hydroxyethyl)phenyl]urea, 1-(3,5-dibromophenyl)-3-[5-chloro-2-(2-hydroxyethyl)phenyl]urea, 1-(3,5-dibromophenyl)-3-[5-fluoro-2-(2-hydroxyethyl)phenyl]urea, 1-(3,5-dibromophenyl)-3-[3,5-dichloro-2-(2-hydroxyethyl)phenyl]urea,
**R1 is Br, R2 is NHetOH:** 1-[3-bromo-5-(2-hydroxyethylamino)phenyl]-3-[2-(2-hydroxyethyl)phenyl]urea, 1-[3-bromo-5-(2-hydroxyethylamino)phenyl]-3-[3-chloro-2-(2-hydroxyethyl)phenyl]urea, 1-[3-bromo-5-(2-hydroxyethylamino)phenyl]-3-[5-chloro-2-(2-hydroxyethyl)phenyl]urea, 1-[3-bromo-5-(2-hydroxyethylamino)phenyl]-3-[5-fluoro-2-(2-hydroxyethyl)phenyl]urea, 1-[3-bromo-5-(2-hydroxyethylamino)phenyl]-3-[3,5-dichloro-2-(2-hydroxyethyl)phenyl]urea,
**R1 is Br, R2 is NHetNH₂:** 1-[3-bromo-5-(2-aminoethylamino)phenyl]-3-[2-(2-hydroxyethyl)phenyl]urea, 1-[3-bromo-5-(2-aminoethylamino)phenyl]-3-[3-chloro-2-(2-hydroxyethyl)phenyl]urea, 1-[3-bromo-5-(2-aminoethylamino)phenyl]-3-[5-chloro-2-(2-hydroxyethyl)phenyl]urea, 1-[3-bromo-5-(2-aminoethylamino)phenyl]-3-[5-fluoro-2-(2-hydroxyethyl)phenyl]urea, 1-[3-bromo-5-(2-aminoethylamino)phenyl]-3-[3,5-dichloro-2-(2-hydroxyethyl)phenyl]urea,
**R1 is Cl, R2 is H**: 1-(3-chlorophenyl)-3-[3-chloro-2-(2-hydroxyethyl)phenyl]urea, 1-(3-chlorophenyl)-3-[5-chloro-2-(2-hydroxyethyl)phenyl]urea, 1-(3-chlorophenyl)-3-[5-fluoro-2-(2-hydroxyethyl)phenyl]urea, 1-(3-chlorophenyl)-3-[3,5-dichloro-2-(2-hydroxyethyl)phenyl]urea,
**R1 is Cl, R2 is F:** 1-(3-chloro-5-fluorophenyl)-3-[2-(2-hydroxyethyl)phenyl]urea, 1-(3-chloro-5-fluorophenyl)-3-[3-chloro-2-(2-hydroxyethyl)phenyl]urea, 1-(3-chloro-5-fluorophenyl)-3-[5-chloro-2-(2-hydroxyethyl)phenyl]urea, 1-(3-chloro-5-fluorophenyl)-3-[5-fluoro-2-(2-hydroxyethyl)phenyl]urea, 1-(3-chloro-5-fluorophenyl)-3-[3,5-dichloro-2-(2-hydroxyethyl)phenyl]urea,
**R1 is Cl, R2 is Cl:** 1-(3,5-dichlorophenyl)-3-[3-chloro-2-(2-hydroxyethyl)phenyl]urea, 1-(3,5-dichlorophenyl)-3-[5-chloro-2-(2-hydroxyethyl)phenyl]urea, 1-(3,5-dichlorophenyl)-3-[5-fluoro-2-(2-hydroxyethyl)phenyl]urea, 1-(3,5-dichlorophenyl)-3-[3,5-dichloro-2-(2-hydroxyethyl)phenyl]urea,
**R1 is Cl, R2 is NHetOH:** 1-[3-chloro-5-(2-hydroxyethylamino)phenyl]-3-[2-(2-hydroxyethyl)phenyl]urea, 1-[3-chloro-5-(2-hydroxyethylamino)phenyl]-3-[3-chloro-2-(2-hydroxyethyl)phenyl]urea, 1-[3-chloro-5-(2-hydroxyethylamino)phenyl]-3-[5-chloro-2-(2-hydroxyethyl)phenyl]urea, 1-[3-chloro-5-(2-hydroxyethylamino)phenyl]-3-[5-fluoro-2-(2-hydroxyethyl)phenyl]urea, 1-[3-chloro-5-(2-hydroxyethylamino)phenyl]-3-[3,5-dichloro-2-(2-hydroxyethyl)phenyl]urea,
**R1 is Cl, R2 is NHetNH₂:** 1-[3-chloro-5-(2-aminoethylamino)phenyl]-3-[2-(2-hydroxyethyl)phenyl]urea, 1-[3-chloro-5-(2-aminoethylamino)phenyl]-3-[3-chloro-2-(2-hydroxyethyl)phenyl]urea, 1-[3-chloro-5-(2-aminoethylamino)phenyl]-3-[5-chloro-2-(2-hydroxyethyl)phenyl]urea, 1-[3-chloro-5-(2-aminoethylamino)phenyl]-3-[5-fluoro-2-(2-hydroxyethyl)phenyl]urea, 1-[3-chloro-5-(2-aminoethylamino)phenyl]-3-[3,5-dichloro-2-(2-hydroxyethyl)phenyl]urea,
**R1 is F, R2 is H:** 1-[3-chloro-2-(2-hydroxyethyl)phenyl]-3-(3-fluorophenyl)urea, 1-[5-chloro-2-(2-hydroxyethyl)phenyl]-3-(3-fluorophenyl)urea, 1-[5-fluoro-2-(2-hydroxyethyl)phenyl]-3-(3-fluorophenyl)-urea, 1-[3,5-dichloro-2-(2-hydroxyethyl)phenyl]- 3-(3-fluorophenyl)urea,
**R1 is F, R2 is F:** 1-[3-chloro-2-(2-hydroxyethyl)phenyl]-3-(3,5-difluorophenyl)urea, 1-[5-chloro-2-(2-hydroxyethyl)phenyl]-3-(3,5-difluorophenyl)urea, 1-[5-fluoro-2-(2-hydroxyethyl)phenyl]-3-(3,5-difluorophenyl)urea, 1-[3,5-dichloro-2-(2-hydroxyethyl)phenyl]-3-(3,5-difluorophenyl)urea,
**R1 is F, R2 is NHetOH:** 1-[3-fluoro-5-(2-hydroxyethylamino)phenyl]-3-[2-(2-hydroxyethyl)phenyl]urea, 1-[3-fluoro-5-(2-hydroxyethylamino)phenyl]-3-[3-chloro-2-(2-hydroxyethyl)phenyl]urea, 1-[3-fluoro-5-(2-hydroxyethylamino)phenyl]-3-[5-chloro-2-(2-hydroxyethyl)phenyl]urea, 1-[3-fluoro-5-(2-hydroxyethylamino)phenyl]-3-[5-fluoro-2-(2-hydroxyethyl)phenyl]urea, 1-[3-fluoro-5-(2-hydroxyethylamino)phenyl]-3-[3,5-dichloro-2-(2-hydroxyethyl)phenyl]urea,
**R1 is F, R2 is NHetNH₂:** 1-[3-fluoro-5-(2-aminoethylamino)phenyl]-3-[2-(2-hydroxyethyl)phenyl]urea, 1-[3-fluoro-5-(2-aminoethylamino)phenyl]-3-[3-chloro-2-(2-hydroxyethyl)phenyl]urea, 1-[3-fluoro-5-(2-aminoethylamino)phenyl]-3-[5-chloro-2-(2-hydroxyethyl)phenyl]urea, 1-[3-fluoro-5-(2-aminoethylamino)phenyl]-3-[5-fluoro-2-(2-hydroxyethyl)phenyl]urea, 1-[3-fluoro-5-(2-aminoethylamino)phenyl]-3-[3,5-dichloro-2-(2-hydroxyethyl)phenyl]urea.

In the most preferred embodiment, the compound of the general formula (I) is selected from the group comprising:
1-(2,6-dichloropyridin-4-yl)-3-(2-hydroxymethylphenyl)urea, 1-(5-chloro-2-hydroxymethylphenyl)-3-(2,6-dichloropyridin-4-yl)urea, 1-(2-methoxypyridin-4-yl)-3-(2-hydroxymethylphenyl)urea, 1-(5-chloro-2-hydroxymethylphenyl)-3-(2-methoxypyridin-4-yl)urea, 1-(2-hydroxymethylphenyl)-3-(3-trifluoromethoxyphenyl)urea, 1-(5-chloro-2-hydroxymethylphenyl)-3-(3-trifluoromethoxyphenyl)urea, 1-(3-chloro-5-trifluoromethoxyphenyl)-3-(2-hydroxymethylphenyl)urea, 1-(3-bromo-5-trifluoromethoxyphenyl)-3-(2-hydroxymethylphenyl)urea, 1-(3-chloro-5-methoxyphenyl)-3-(2-hydroxymethylphenyl)urea, 1-(3 -bromo-5 -methoxyphenyl)-3 -(2-hydroxymethylphenyl)urea, 1-(3-bromo-5-chlorophenyl)-3-(2-hydroxymethylphenyl)urea, 1-(2,6-dichloropyridin-4-yl)-3-[2-(2-hydroxyethyl)phenyl]urea, 1-[2-(2-hydroxyethyl)phenyl]-3-(3-trifluoromethoxyphenyl)urea, 1-(3-chloro-5-methoxyphenyl)-3-[2-(2-hydroxyethyl)phenyl]urea, 1-(3-bromo-5-methoxyphenyl)-3-[2-(2-hydroxyethyl)phenyl]urea, 1-(3,5-dichlorophenyl)-3-[2-(2-hydroxyethyl)phenyl]urea.

The compounds of the general formula (I) according to the present invention effectively increase endogenous cytokinin levels in plants, which leads to their higher viability under both optimal and stress conditions, and to their higher yields.

Another object of the present invention is the use of compounds of the general formula (Ia), 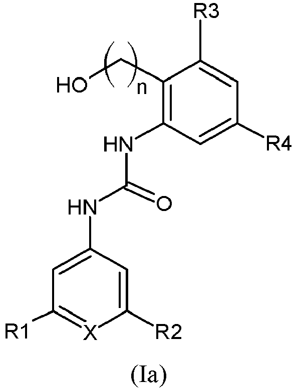 wherein
n is 1 or 2;
X is nitrogen atom (N) or carbon atom (C);
R1 is selected from the group consisting of hydrogen, halogen, methoxy group, trifluoromethoxy group, methylsulphanyl group or trifluoromethylsulphanyl group;
R2 is selected from the group consisting of hydrogen, halogen, amino group, C₁-C₅ alkyl, C₂-C₅ alkenyl, C₂-C₅ alkynyl, and C₁-C₅ alkylamino group, wherein C₁-C₅ alkyl, C₂-C₅ alkenyl, C₂-C₅ alkynyl and C-C₅ alkylamino group may optionally be substituted with hydroxy and/or amino group;
R3 and R4 are independently hydrogen or halogen;
with the proviso that when X is nitrogen, then R2 is hydrogen or halogen;
in agriculture to delay chlorophyll degradation and plant tissue senescence. These effects increase the viability of plants under stress conditions and thus increase crop yields.

In one preferred embodiment of the use of the compound of the general formula (Ia) in agriculture, is n = 1 (hydroxymethyl).

In one preferred embodimentof the use of the compound of the general formula (Ia) in agriculture, R1 is selected from the group consisting of hydrogen, fluorine, chlorine, bromine, methoxy group, trifluoromethoxy group, methylsulphanyl group or trifluoromethylsulphanyl group.

In one preferred embodimentof the use of the compound of the general formula (Ia) in agriculture, R2 is selected from the group consisting of hydrogen, fluorine, chlorine, bromine, amino group, 2-aminoethylamino group and 2-hydroxyethylamino group.

In one preferred embodiment of the use of the compound of the general formula (Ia) in agriculture, R3 and R4 are independetly selected from the group consisting of hydrogen, fluorine, chlorine, bromine. Preferably, R4 is fluorine, chlorine or bromine and R3 is hydrogen.

In one preferred embodimentof the use of the compound of the general formula (Ia) in agriculture, is n = 1, R1 is selected from the group consisting of fluorine, chlorine, bromine, methoxy group, trifluoromethoxy group, methylsulphanyl group or trifluoromethylsulphanyl group; R2 is selected from the group consisting of hydrogen, fluorine, chlorine, bromine, amino group, 2-aminoethylamino group and 2-hydroxyethylamino group; R3 and R4 are independetly selected from the group consisting of hydrogen, fluorine, chlorine, and bromine atom.

In one preferred embodiment of the use of the compound of the general formula (Ia) in agriculture, the compound of the general formula (Ia) is selected from the group comprising: 1-(2,6-dichloropyridin-4-yl)-3-(2-hydroxymethylphenyl)urea, 1-(5-chloro-2-hydroxymethylphenyl)-3-(2,6-dichloropyridin-4-yl)urea, 1-(2-methoxypyridin-4-yl)-3-(2-hydroxymethylphenyl)urea, 1-(5-chloro-2-hydroxymethylphenyl)-3-(2-methoxypyridin-4-yl)urea, 1-(2-hydroxymethylphenyl)-3-(3-trifluoromethoxyphenyl)urea, 1-(5-chloro-2-hydroxymethylphenyl)-3-(3-trifluoromethoxyphenyl)urea, 1-(3-chloro-5-trifluoromethoxyphenyl)-3-(2-hydroxymethylphenyl)urea, 1-(3-bromo-5-trifluoromethoxyphenyl)-3-(2-hydroxymethylphenyl)urea, 1-(3-chloro-5-methoxyphenyl)-3-(2-hydroxymethylphenyl)urea, 1-(3 -bromo-5 -methoxyphenyl)-3 -(2-hydroxymethylphenyl)urea, 1-(3-bromo-5-chlorophenyl)-3-(2-hydroxymethylphenyl)urea, 1-(2,6-dichloropyridin-4-yl)-3-[2-(2-hydroxyethyl)phenyl]urea, 1-[2-(2-hydroxyethyl)phenyl]-3-(3-trifluoromethoxyphenyl)urea, 1-(3-chloro-5-methoxyphenyl)-3-[2-(2-hydroxyethyl)phenyl]urea, 1-(3-bromo-5-methoxyphenyl)-3-[2-(2-hydroxyethyl)phenyl]urea, 1-(3,5-dichlorophenyl)-3-[2-(2-hydroxyethyl)phenyl]urea, 1-(2,6-dichloropyridin-4-yl)-3-[2-(2-hydroxyethyl)phenyl]urea, 1-[2-(2-hydroxyethyl)phenyl]-3-(3-trifluoromethoxyphenyl)urea, 1-[2-(2-hydroxyethyl)phenyl]-3-(3-methoxyphenyl)urea, 1-(3,5-dichlorophenyl)-3-[2-(2-hydroxyethyl)phenyl]urea.

The compounds of the present invention of the general formula (Ia) for use in agriculture, can be applied to whole plants, plant organs, or to plant cells and plant tissues. The compounds of the general formula (Ia) according to the present invention are also suitable for use in plant tissue cultures, where they prevent the degradation of cytokinins and thus increase the viability of new plants, regeneration of buds and shoots from explants, callus growth, suppress apical dominance, reduce hyperhydricity of plants grown *in vitro* and show very strong micropropagation activity.

A further aspect of the invention is the use of the substituted 1-[2-(hydroxyalkyl)phenyl]-3-ylurea derivatives of the general formula (Ia) as inhibitors of stress and stress-induced senescence in the production of crops, in particular to increase the yield and quality of agricultural products in adverse conditions, such as drought, heat, salinity, strong light, cold, frost and floods. The agriculturally important crops can be selected from in particular cereals (wheat, barley, rice, maize, rye, oat, sorghum, and related species), beet (sugar beet and fodded beet); pomes, drupes and soft fruits (apples, pears, plums, peaches, almonds, cherries, strawberries and blackberries); leguminous plants (beans, lentils, peas, soybeans); oil plants (rape, mustard, poppy, olives, sunflowers, coconut, Ricinus, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, cinnamomum, camphor) or plants such as tobacco, nuts, eggplants, sugar cane, tea, vine gpapes, hops, bananas and natural rubber and medicinal plants, as well as ornamentals. Crops include those which have been rendered tolerant towards classes of growth factors by conventional breeding methods or genetic engineering methods. The compounds of the general formula (Ia) accelerate the pouring of seeds and the increase in the size of seeds and fruits of plants, shorten the germination time of seeds, increase yields and quality of agricultural products, and finally increase resistance to environmental stresses.

The present invention further includes antisenescent and/or antistress preparations for plants, plant organs and plant cells, comprising at least one compound of the general formula (I) and at least one auxiliary substance (excipients). The auxiliary substances can be in solid or liquid state. Excipient refers to solvents, solid carriers, surface active agents (surfactants), emulsifiers, dispersants, wetting agents, stabilizers, defoamers, preservatives, viscosity agents, adhesives, and also fertilizers or other active ingredients.

Suitable solvents are, for example, aromatic hydrocarbons having a carbon number of 8 to 12 (e.g. xylene or naphthalene derivatives), phthalates (for example dibutyl phthalate, dioctyl phthalate), aliphatic hydrocarbons (e.g. cyclohexane, paraffin), alcohols, glycols and their ethers and esters (e.g. ethanol, ethylene glycol, 2- methoxyethanol, 2-ethoxyethanol), ketones (e.g. cyclohexanone, N-methyl-2-pyrrolidone, DMSO, DMF), vegetable oils, water.

Solid carriers are typically calcite, talc, kaolin, montmorillonite or attapulgite, silicic acid, polymers, crushed limestone.

Suitable surfactants are nonionic, cationic or anionic surfactants (alkali metal salts, alkaline earth metal salts or ammonium salts of higher fatty acids having a carbon number of 10 to 20, especially sodium or potassium salts of oleic or stearic acid, sulfonated fatty acids, sulfonated benzimidazole derivatives, alkyl sulfonates, phosphoric acid esters). Nonionic surfactants are typically derivatives of polyglycol ether and aliphatic or cycloaliphatic alcohols or saturated or unsaturated fatty acids and alkylphenols, water-soluble adducts of polyethylene oxide with polyethylene glycol.

Stabilizers are, for example, vegetable oils or epoxidized vegetable oils (epoxidized palm oil, rapeseed or olive oil).

The defoamer is, for example, silicone oil.

Examples of suitable anionic, non-ionic and cationic surfactants are listed, for example, in WO 97/34485.

The invention further relates to a method for inhibiting stress and/or senescence in plants, plant organs and/or plant cells, which comprises applying at least one compound of the general formula (I) or (Ia) to a plant, plant organ and/or plant cell.

The compounds of the general formula (I) and (Ia) of the present invention can be prepared by a method in which an aniline (or pyridine-4-amine) bearing R1 and/or R2 is converted to an isocyanate by a conventional method using diphosgene (Kurita K. et al, (1976) J. Org. Chem. 41, 2070-71). The resulting isocyanate then reacts with an aniline bearing a hydroxyalkyl group to give the desired product. To increase the yield, the hydroxyl group may be protected with a suitable protecting group. This protecting group must be removed after reaction with the isocyanate.

The compounds of the general formula (I) and (Ia) are used in unmodified form or, preferably, together with excipients conventionally employed in the art of preparations. To this end they are conveniently formulated as concentrates of active compounds as well as suspensions and dispersions, preferentially isotonic water solutions, suspensions and dispersion, diluted emulsions, soluble powders, dusts, granulates, creams, gels, oil suspensions and also encapsulations, e.g. in polymeric substances. As with the type of the preparation, the methods of application, such as spraying, atomizing, dusting, scattering, coating or pouring, are chosen in accordance with the intended objectives and the prevailing circumstances. The preparations may be sterilized and/or contain further excipients of neutral nature such as preservatives, stabilizers, wetting agents or emulgators, solubilizing agents, as well as fertilizers, micronutrient donors or other formulations for obtaining special effects.

### Preparations

The preparations comprising the compounds of general formula I or (Ia) (active ingredients) and, where appropriate, one or more solid or liquid excipients, are prepared in a manner known per se e.g. by mixing and/or grinding the active ingredients with excipients, e.g. solvents or solid carriers. In addition, surface-active compounds (surfactants) may also be used in the preparations. Depending on the nature of the compound of general formula (I) or (Ia) to be formulated, suitable surface-active compounds are non-ionic, cationic and/or anionic surfactants and surfactant mixtures having good emulsifying, dispersing and wetting properties.

Examples of suitable anionic, non-ionic and cationic surfactants are listed, for example, in WO 97/34485.

Also suitable in the preparation of the compositions containg compounds of the general formula (I) or (Ia) according to the invention are the surfactants conventionally used in formulation technology, which are described, *inter alia,* in "McCutcheon's Detergents and Emulsifiers Annual" MC Publishing Corp., Ridgewood New Jersey, 1981; Stache, H., "Tensid-Taschenbuch", Carl Hanser Verlag, Munich, 1981; and M. and J. Ash, "Encyclopedia of Surfactants", Vol.l-3, Chemical Publishing Co., New York, 1980-81.

The formulation of the preparation contains from 0.01 to 99.09 % (w/w), in particular from 0.1 to 95 % (w/w), of the active ingredient corresponding to the compound or mixture of compounds of the general formula (I) or (Ia), and from 0.01 to 99.09 % (w/w) of a mixture of additives or other carriers, depending on the methods of application, preferably from 5 to 99.9 % (w/w) of a mixture of additives or other carriers; and optionally may further comprise from 0.1 to 40 % (w/w) of a wetting agent, preferably from 0.5 to 30 % (w/w) of a wetting agent, more preferably from 1 to 20 % (w/w) of a wetting agent.

Whereas commercial products are usually formulated as concentrates, the end user will normally employ dilute formulations. The compositions may also comprise further ingredients, such as stabilisers, e.g. vegetable oils or epoxidised vegetable oils (epoxidised palm oil, coconut iol, rapeseed oil or soybean oil), antifoams, e.g. silicone oil, preservatives, stabilizers, wetting agents or emulsifiers, viscosity factors, binders, tackifiers, and also fertilisers or other active ingredients. Preferred formulations have the following compositions: (% = percent by weight)
Emulsifiable concentrates:
   active ingredient mixture: 0.01 to 90 %, surfactant: 1 to 30 %, liquid carrier: 5 to 94 %
Dusts:
   active ingredient mixture: 0.1 to 10 %, solid carrier: 99.9 to 90 %,
Suspension concentrates:
   active ingredient mixture: 0.5 to 75 %, water: 94 to 24 %, surfactant: 1 to 40 %,
Wettable powders:
   active ingredient mixture: 0.5 to 90 %, surfactant: 0.5 to 20 %, solid carrier: 5 to 95 %,
Granules:
   active ingredient mixture: 0.1 to 30 %, solid carrier: 99.9 to 70 %.

The compositions may also comprise further ingredients, such as stabilisers, e.g. vegetable oils or epoxidised vegetable oils (epoxidised coconut oil, rapeseed oil or soybean oil), anti-foams, e.g. silicone oil, preservatives, viscosity regulators, binders, tackifiers, and also fertilisers or other active ingredients. For the use of the compounds of general formula (I) or (Ia), or of compositions comprising them, in the protection of crop plants against the damaging effects of growth regulators, various methods and techniques come into consideration, such as, for example, the following:
i) Seed dressing
   a) Dressing of the seeds with a wettable powder formulation of a compound of the general formula (I) or (Ia) by shaking in a vessel until uniformly distributed over the seed surface (dry dressing). In that procedure approximately from 1 to 500 g of compound of the general formula (I) or (Ia) (4 g to 2 kg of wettable powder) are used per 100 kg of seed.
   b) Dressing of the seeds with an emulsifiable concentrate of a compound of formula (I) or (Ia) according to method a) (wet dressing).
   c) Dressing by immersing the seeds for from 1 to 72 hours in a liquor comprising from 100 to 1000 ppm of a compound of general formula (I) or (Ia) and preferably subsequently drying the seeds (immersion dressing).
   Dressing the seeds or treating the germinated seedlings are naturally the preferred methods of application, because treatment with the active ingredients is directed entirely at the target crop. Generally from 1 to 1000 g of antidote, preferably from 5 to 250 g of antidote, are used per 100 kg of seed, but depending on the methodology, which also enables the addition of other active ingredients or micronutrients:, the concentration limits indicated can be varied up or down (repeat dressing).
ii) Application as a tank mixture
   A liquid formulation is used in the amount of 0.005 to 5.0 kg per hectare. Such tank mixtures are applied before or after sowing.
iii) Application to the seed furrow
   The compounds of formula (I) or (Ia) are introduced into the open, sown seed furrow in the form of an emulsifiable concentrate, wettable powder or granules. Once the seed furrow has been covered over, the growth regulator is applied in the usual manner in the pre-emergence process.
iv) Controlled release of active ingredient
   The compounds of formula (I) or (Ia) are applied in solution to mineral granule carriers or polymerised granules (urea/formaldehyde) and dried. If desired, it is also possible to apply a coating that allows the active ingredient to be released in metered amounts over a specific period of time (coated granules).

The method of preparation of the compounds of the general formula (I) or (Ia) is based on the reaction of 1 equivalent of substituted pyridin-4-yl isocyanate or substituted isocyanatobenzene with 1 equivalent of substituted aniline according to Scheme 1 (step B). Commercially unavailable isocyanates are prepared by reacting a substituted pyridin-4-amine or a substituted aniline with diphosgene in THF (Kurita K. et al, (1976) J. Org. Chem. 41, 2070-71) according to Scheme 1 (step A).

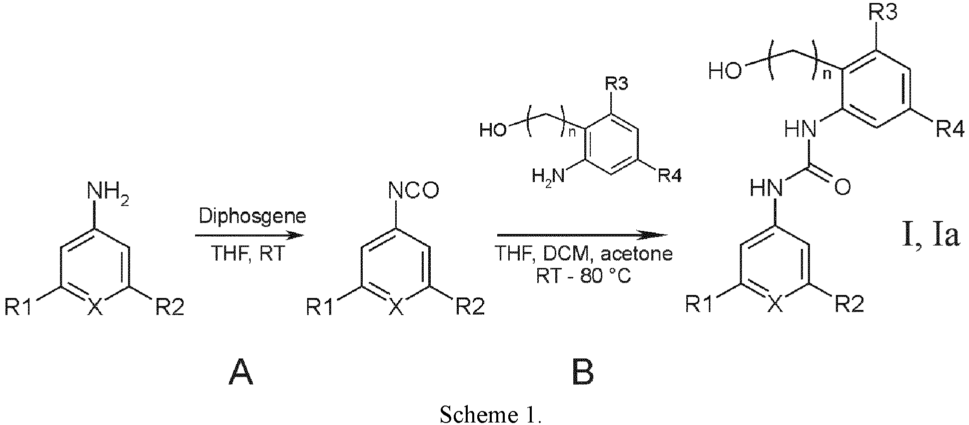

### Brief description of drawings

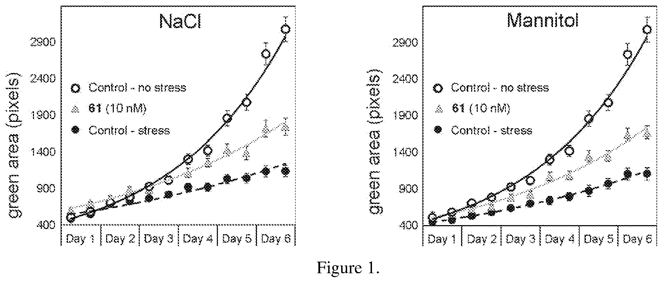

Figure 1 shows the effect of compound **61** on the reduction of growth, which was caused by two stress factors.

### Examples

All compounds falling under the scope of the general formula (Ia) were prepared by reacting 1 equivalent of substituted 4-isocyanatopyridine or substituted isocyanatobenzene with 1 equivalent of substituted aniline according to Scheme 1 (step B). Commercially available isocyanates used in the present invention: 2,6-dichloro-4-isocyanatopyridine, 1,3-difluoro-5-isocyanatobenzene, 1,3-dichloro-5-isocyanatobenzene, 1-isocyanato-3-(trifluoromethoxy)benzene, 1-isocyanato-3-(trifluoromethylsulphanyl)benzene.

If the isocyanate was not commercially available, it was prepared by the known reaction of substituted pyridine-4-amine or substituted aniline with diphosgene in THF (Kurita K. a Iwakura Y., (1976) J. Org. Chem. 41, 2070-71) according to Scheme 1 (Step A, Example 1). The following commercially available amines were used for this purpose: 2-fluoropyridin-4-amine, 2,6-difluoropyridin-4-amine, 2-chloropyridin-4-amine, 2-methoxypyridin-4-amine, 3,5-dibromoaniline, 3-bromo-5-fluoroaniline, 3-bromo-5-chloroaniline, 3-fluoro-5-methoxyaniline, 3-chloro-5-methoxyaniline, 3-bromo-5-methoxyaniline, 3-(methylthio)aniline, 3-chloro-5-(trifluormethoxy)aniline, 3-bromo-5-(trifluormethoxy)aniline.

The isocyanates prepared from the above amines were used in crude form for further syntheses (Scheme 1, step B). Most of the anilines for the preparation of isocyanates are commercially available. Some anilino-alcohols that we have reacted with isocyanates have not yet been prepared. Their synthesis is described below.

### Example 1. Preparation of isocyanates

One equivalent of substituted pyridin-4-amine or substituted aniline was dissolved in dry THF and mixed with 2 equivalents of triethylamine. This mixture was slowly added dropwise to a solution of diphosgene (0.6 equivalent) in THF at -20 °C. The temperature of the reaction mixture (RM) was then brought to room teperature (r.t.) or higher (about 60 °C). The precipitated triethylamine hydrochloride was filtered off and the filtrate was evaporated to dryness. The crude isocyanate remained in the reaction vessel and was used for the following synthesis.

### Example 2. 1-(2-Chloropyridin-4-yl)-3-[2-(2-hydroxyethyl)phenyl]urea (12)

All aromatic amines used in the present invention contain a hydroxyl group. This group was protected with a tert-butyldimethylsilyl group prior to the reaction of this aromatic amine with a substituted 4-isocyanatopyridine or isocyanatobenzene. Deprotection was performed in propanolic HCl, all according to Greene TW and Wuts PGM; Protective Groups in Organic Synthesis 3rd ed., New York, (1991). A detailed description of the method is given below. This method was used for the synthesis of all other compounds of this invention.

2-(2-Aminophenyl)-ethanol (274 mg, 2.0 mmol) was dissolved in dry THF and triethylamine (0.5 mL, 3.0 mmol), *tert*-butyldimethylsilyl chloride (350 mg, 2.2 mmol) and dimethylaminopyridine (5 mg, catalytic amount) were added. The RM was stirred overnight at r.t. The next day, triethylamine hydrochloride was filtered off and the THF was evaporated to dryness. The liquid product was partitioned between water and ethyl acetate. The ethyl acetate part was collected, dried with MgSO₄ and evaporated to dryness. The product was a pink liquid 2-[2-(*tert-*butyldimethylsilanyloxy)ethyl]aniline **(12a).**

2-Chloro-4-isocyanatopyridine (127 mg, 0.5 mmol) was dissolved in THF and **12a** (126 mg, 0.5 mmol) was added with stirring. The RM was stirred for 1 h at r.t. Then TLC indicated the formation of the protected product and the absence of starting materials. The THF was then evaporated to dryness and ethanol (10 mL) and 0.3 mL of 35% HCl were added. The deprotection of the OH group was monitored by TLC analysis (mobile phase CHCl₃). After OH-deprotection, the solvent was evaporated to dryness several times with the addition of ethanol. Finally, the product was resuspended in water, filtered off and crystallized from ethanol (optionally from a mixture of ethanol, water). Yield 50 mg, 34 %. **HPLC:** purity 98.5 %, *R*ₜ = 22.07 min.; **¹H NMR** (500 MHz, DMSO-*d₆*): δ 2.71 (2H, *t, J*=6.5 Hz, CH₂), 3.58 (2H, *q, J₁*=4.5 Hz, *J₂*=7.0 Hz, CH₂), 4.90 (1H, *t, J*=4.5 Hz, OH), 7.02 (1H, *t, J*=7.0 Hz, ArH), 7.15 (1H, *t, J*=8.0 Hz, ArH), 7.18 (1H, *d, J*=7.5 Hz, ArH), 7.25 (1H, *d, J*=5.5 Hz, ArH), 7.60 (1H, *d, J*=7.5 Hz, ArH), 7.63 (1H, *ds, J*=1.5 Hz, ArH), 8.13 (1H, *d, J*=5.5 Hz, ArH), 8.33 (1H, *s*, NH), 9.75 (1H, *s*, NH); **MS-ESI⁺** Coin Voltage (CV) 20 V, *m*/*z* (rel. int.): 292.0 [M+H]⁺ (100), 294.0 [M+H]⁺ (60); **EM** 291.

The same procedure for protection and deprotection of the hydroxy group was used for all other substances.

### Example 3. 1-(2,6-Dichloropyridin-4-yl)-3-[2-(2-hydroxyethyl)phenyl]urea (13)

2,6-Dichloro-4-isocyanatopyridin (95 mg, 0.5 mmol) was dissolved in THF and **12a** (126 mg, 0.5 mmol) was added with stirring. The RM was stirred for 1 h at r.t. The product was obtained according to Example 2. Yield 81 mg, (50 %). **HPLC:** purity 99.25 %, *R*ₜ = 22.9 min.; **¹H NMR** (500 MHz, DMSO-*d₆*): δ 2.70 (2H, *t, J*=6.5 Hz, CH₂), 3.58 (2H, *q, J₁*=4.5 Hz, *J₂*=7.0 Hz, CH₂), 4.95 (1H, *t, J*=4.5 Hz, OH), 7.04 (1H, *t, J*=7.5 Hz, ArH), 7.15 (1H, *t, J*=7.5 Hz, ArH), 7.19 (1H, *d, J*=7.5 Hz, ArH), 7.51 (2H, s, ArH), 7.56 (1H, *d, J*=8.0 Hz, ArH), 8.47 (1H, *s,* NH), 9.96 (1H, *s,* NH); **MS-ESI⁺** CV 20 V, *m*/*z* (rel. int.): 326.0 [M+H]⁺ (100), 328.0 [M+H]⁺ (80); **EM** 325.

**Example 4.** 1-(3,5-Dichlorophenyl)-3-(2-hydroxymethylphenyl)urea **(18)** is diclaimed under the provisio in claim 1 and thus it is out of scope of this invention and it only serves for explanation. 1,3-Dichloro-5-isocyanatobenzene (94 mg, 0.5 mmol) was dissolved in THF and 2-(*tert-*butyldimethylsilanyloxymethyl)aniline was added with stirring (118 mg, 0.5 mmol). The RM was stirred for 1 h at r.t. The product was obtained according to Example 2. Yield 141 mg, 91%). **HPLC:** purity 98.6 %, *R*ₜ = 25.0 min.; **¹H NMR** (500 MHz, DMSO-*d₆*): δ 4.48 (2H, s, CH₂), 5.45 (1H, *s* (br), OH), 7.0 (1H, *t, J*=7.5 Hz, ArH), 7.10 (1H, s, ArH), 7.20 (1H, *t, J*=7.5 Hz, ArH), 7.26 (1H, *d, J*=7.0 Hz, ArH), 7.50 (2H, s, ArH), 7.76 (1H, *d, J*=8.0 Hz, ArH), 8.31 (1H, *s,* NH), 9.72 (1H, *s,* NH); **MS-ESI⁺** CV 20 V, *m*/*z* (rel. int.): 292.9 [M-OH]⁺ (40), 294.7 [M-OH]⁺ (30), 311.0 [M+H]⁺ (30), 313.0 [M+H]⁺ (25), 332.8 [M+Na]⁺ (100), 334.7 [M+Na]⁺ (70); **MS-ESI⁻** CV 20 V, *m*/*z* (rel. int.): 308.8 [M-H]⁻ (70), 344.8 [M+Cl]⁻ (80), 346.7 [M+Cl]⁻ (100); **EM** 310.

### Example 5. 1-(3-Chloro-2-hydroxymethylphenyl)-3-(3,5-dichlorophenyl)urea (20)

1,3-Dichloro-5-isocyanatobenzene (75 mg, 0.4 mmol) was dissolved in THF and 2-(*tert-*butyldimethylsilanyloxymethyl)-3-chloroaniline (136 mg, 0.5 mmol) was added. The product was obtained according to Example 2. Yield 50 mg, 33 %). **¹H NMR** (500 MHz, DMSO-*d₆*): δ 4.67 (2H, d, *J*=2.8 Hz, CH2), 5.64 (1H, t (br), OH), 7.11 (1H, d, *J*=8.0 Hz, ArH), 7.13 (1H, t, *J*=1.7 Hz, ArH), 7.25 (1H, t, *J*=8.6 Hz, ArH), 7.50 (2H, d, *J*=2.3 Hz, ArH), 7.80 (1H, d, *J*=7.5 Hz, ArH), 8.57 (1H, s, NH), 9.91 (1H, s, NH); **MS-ESI⁺** CV 20 V, *m*/*z* (rel. int.): 345.0 and 347.0 [M+H]⁺ (80 and 80), 367.0 and 369.0 [M+Na]⁺ (100 and 100), 713.0 and 715.0 [2M+Na]⁺ (50 and 40); **MS-ESI⁻**CV 20 V, *m*/*z* (rel. int.): 343.0 and 344.9 [M-H]⁻ (100 and 80), 378.8 and 380.8 [M+Cl]⁻ (70 and 60); **EM** 344.

### Example 6. 1-(3,5-Dichloro-2-hydroxymethylphenyl)-3-(3,5-dichlorophenyl)urea (21)

2-Amino-4,6-dichlorobenzoic acid (70 mg, 0.34 mmol) was dissolved in THF (10 mL) and this solution was cooled to 0 °C. This solution was bubbled with diborane for 30 minutes, then the solution was transferred to a high pressure tube and the RM was stirred at 60 °C for 15 h. After the reaction, 1 mL of saturated NaHCO₃ solution was added to the RM. The THF was then evaporated and the residue was partitioned between water and ethyl acetate. Evaporation of ethyl acetate gave crude (2-amino-4,6-dichlorophenyl) -methanol, which was converted to 2-(*tert-*butyldimethylsilanyloxymethyl)-3,5-dichloroaniline according to Example 2. This was added to the solution of 1,3-Dichloro-5-isocyanatobenzene (64 mg, 0.34 mmol) in acetone and the RM was stirred at 40 °C overnight. The RM was then worked up as in Example 2. The pure product was crystallized from chloroform. Yield 35 mg (27 %). **¹H NMR** (500 MHz, DMSO-*d₆*): δ 4.65 (2H, d, *J*=5.7 Hz, CH2), 5.73 (1H, t, *J*=5.7 Hz, OH), 7.17 (1H, t, *J*=1.7 Hz, ArH), 7.25 (1H, d, *J*=2.3 Hz, ArH), 7.50 (2H, d, *J*=1.7 Hz, ArH), 7.98 (1H, d, *J*=2.3 Hz, ArH), 8.72 (1H, s, NH), 10.02 (1H, s, NH); **MS-ESI⁺** CV 15 V, *m*/*z* (rel. int.): 379.0 [M+H]⁺ (100); **MS-ESI⁻** CV 15 V, *m*/*z* (rel. int.): 377.1 [M-H]⁻ (100); **EM** 378.

### Example 7. 1-(2-Hydroxymethylphenyl)-3-(3-trifluoromethoxyphenyl)urea (28)

1-Isocyanato-3-(trifluoromethoxy)benzen (102 mg, 0.5 mmol) was dissolved in THF and 2-(*tert-*butyldimethylsilanyloxymethyl)aniline (118 mg, 0.5 mmol) was added with stirring. The RM was stirred for 1 h at r.t. The product was obtained according to Example 2. Yield (55%). **HPLC:** purity 99.9 %, *R*ₜ = 24.1 min.; **¹H NMR** (500 MHz, DMSO-*d₆*): δ 4.47 (2H, d, *J*=6.0 Hz, CH₂), 5.40 (1H, t, *J*=5.0 Hz, OH), 6.88 (1H, d, *J*=7.0 Hz, ArH), 6.98 (1H, t, *J*=7.5 Hz, ArH), 7.19 (1H, t, *J*=7.5 Hz, ArH), 7.26 (2H, t, *J*=7.5 Hz, ArH), 7.34 (1H, t, *J*=8.0 Hz, ArH), 7.65 (1H, s, NH), 7.70 (1H, d, *J*=7.5 Hz, ArH), 8.21 (1H, s, NH). **MS-ESI⁺** CV 15 V, *m*/*z* (rel. int.): 326.9 [M+H]⁺ (100). **MS-ESI⁻** CV 15 V, *m*/*z* (rel. int.): 325.0 [M-H]⁻ (100), 651 [2M-H]⁻ (30); **EM** 326.

### Example 8. 1-(3-fluoro-2-hydroxymethylphenyl)-3-(3-trifluoromethoxyphenyl)urea (29)

1-Isocyanato-3-(trifluoromethoxy)benzen (102 mg, 0.5 mmol) was dissolved in THF and 2-(*tert-*butyldimethylsilanyloxymethyl)-3-fluoroaniline (0.5 mmol) was added with stirring. The RM was stirred for 1 h at r.t. The crude product was obtained according to Example 2 and recrystalized from chloroform. White crystal, yield 40 mg (30 %). **¹H NMR** (500 MHz, DMSO-*d₆*): δ 4.55 (2H, s, CH₂), 5.49 (1H, s (br), OH), 6.82 (1H, t, *J*=8.6 Hz, ArH), 6.91 (1H, d, *J*=8.0 Hz, ArH), 7.24 (1H, q, *J*=7.6 Hz, ArH), 7.28 (1H, d, *J*=8.3 Hz, ArH), 7.36 (1H, t, *J*=8.0 Hz, ArH), 7.66, 1H, s, ArH), 7.74 (1H, d, *J*=8.3 Hz, ArH), 8.43 (1H, s, NH), 9.84 (1H, s, NH). **MS-ESI⁺** CV 20 V, *m*/*z* (rel. int.): 327.0 [M-H₂O]⁺ (20), 345.0 [M+H]⁺ (70), 367.0 [M+Na]⁺ (40), 689.1 [2M+H]⁺ (50), 711.1 [2M+Na]⁺ (100). **MS-ESI⁺** CV 40 V, *m*/*z* (rel. int.): 327.0 [M-H₂O]⁺ (100), 345.0 [M+H]⁺ (30), 367.0 [M+Na]⁺ (90); **EM** 344.

### Example 9. 1-(3-Chloro-2-hydroxymethylphenyl)-3-(3-trifluoromethoxyphenyl)urea (30)

1-Isocyanato-3-(trifluoromethoxy)benzen (102 mg, 0.5 mmol) was dissolved in THF and 2-(*tert-*butyldimethylsilanyloxymethyl)-3-chloroaniline (0.5 mmol) was added with stirring. The RM was stirred at 55 °C for 3 h and then overnight at r.t. The crude product was obtained according to Example 2 and recrystalized from chloroform. White crystal, yield 54 mg (30 %). **¹H NMR** (500 MHz, DMSO-*d₆*): δ 4.67 (2H, d, *J*=5.7 Hz, CH2), 5.58 (1H, t, *J*=5.7 Hz, OH), 6.91 (1H, d, *J*=8.0 Hz, ArH), 7.10 (1H, d, *J*=8.0 Hz, ArH), 7.24 (1H, t, *J*=8.0 Hz, ArH), 7.29 (1H, d, *J*=7.5 Hz, ArH), 7.36 (1H, t, *J*=8.0 Hz, ArH), 7.66 (1H, s, ArH), 7.82 (1H, d, *J*=7.5 Hz, ArH), 8.51 (1H, s, NH), 9.84 (1H, s, NH); **MS-ESI⁺** CV 25 V, *m*/*z* (rel. int.): 343.0 and 345.0 [M-H₂O]⁺ (100 and 30), 383.0 and 385.0 [M+Na]⁺ (60 and 20); **MS-ESI⁻** CV 20 V, *m*/*z* (rel. int.): 358.9 [M-H]⁻ (100); **EM** 360.

### Example 10. 1-(5-fluoro-2-hydroxymethylphenyl)-3-(3-trifluoromethoxyphenyl)urea (32)

1-Isocyanato-3-(trifluoromethoxy)benzen (102 mg, 0.5 mmol) was dissolved in THF and 2-(*tert-*butyldimethylsilanyloxymethyl)-5-fluoroaniline (0.5 mmol). The RM was stirred overnight at r.t. The crude product was obtained according to Example 2. The pure product was obtained by column chromatography (CC) (mobile phase - chloroform). White powder, yield 30 mg (17 %). **¹H NMR** (500 MHz, DMSO-*d₆*): δ 4.47 (2H, d, *J*=5.7 Hz, CH₂), 5.45 (1H, t, *J=5.5* Hz, OH), 6.78 (1H, dt, *Jₜ*=8.6 Hz, *J_{d}*=2.9 Hz, ArH), 6.92 (1H, td, *J_{d}*=8.0 Hz, *Jₜ*=1.2 Hz, ArH), 7.26 (1H, d, *J*=6.9 Hz, ArH), 7.26 (1H, d, *J*=6.9, ArH), 7.29 (1H, d, *J*=7.5 Hz, ArH), 7.37 (1H, t, *J*=8.0 Hz, ArH), 7.65 (1H, s, ArH), 7.77 (1H, dd, *J₁*=12.0 Hz, *J₂*=2.9 Hz, ArH), 8.39 (1H, s, NH), 9.78 (1H, s, NH). **MS-ESI⁺** CV 20 V, *m*/*z* (rel. int.): 345.0 [M+H]⁺ (20), 367.0 [M+Na]⁺ (100), 711.0 [2M+Na]⁺ (20). **MS-ESI⁻** CV 20 V, *m*/*z* (rel. int.): 342.9 [M-H]⁻ (100), 687.0 [2M-H]⁻ (20); **EM** 344.

### Example 11. 1-(5-Chloro-2-hydroxymethylphenyl)-3-(3-trifluoromethoxyphenyl)urea (33)

1-Isocyanato-3-(trifluoromethoxy)benzen (102 mg, 0.5 mmol) was dissolved in THF and 2-(*tert-*butyldimethylsilanyloxymethyl)-5-bromoaniline (0.5 mmol) was added with stirring. The product was obtained according to Example 2. White crystal, yield 63 mg (35 %). **¹H NMR** (500 MHz, DMSO-*d₆*): δ 4.47 (2H, d, *J*=5.7 Hz, CH₂), 5.50 (1H, t, *J*=5.0 Hz, OH), 6.92 (1H, d, *J*=8.0 Hz, ArH), 7.03 (1H, dd, *J₁*=8.0 Hz, *J₂*=1.7 Hz, ArH), 7.28 (2H, d, *J*=8.0 Hz, ArH), 7.37 (1H, t, *J*=8.0 Hz, ArH), 7.66 (1H, s, ArH), 7.98 (1H, d, *J*=2.3 Hz, ArH), 8.35 (1H, s, NH), 9.75 (1H, s, NH). **MS-ESI⁺** CV 20 V, *m*/*z* (rel. int.): 343.0 and 345.0 [M-H₂O]⁺ (100 and 35), 361.0 and 363.1 [M+H]⁺ (30 and 10), 383.0 [M+Na]⁺ (30). **MS-ESI⁺** CV 8 V, *m*/*z* (rel. int.): 343.0 and 345.0 [M-H₂O]⁺ (40 and 15), 361.0 and 363.1 [M+H]⁺ (100 and 35), 383.0 [M+Na]⁺ (30). **MS-ESI⁻** CV 20 V, *m*/*z* (rel. int.) with NH₄OH: 359.0 and 361.0 [M-H]⁻ (100 and 35); **EM** 360.

### Example 12. 1-(5-Bromo-2-hydroxymethylphenyl)-3-(3-trifluoromethoxyphenyl)urea (34)

1-Isocyanato-3-(trifluoromethoxy)benzen (102 mg, 0.5 mmol) was dissolved in THF and 2-(*tert-*butyldimethylsilanyloxymethyl)-5-bromoaniline (0.5 mmol) was added with stirring. The product was obtained according to Example 2. White crystal, yield 101 mg (50 %). **¹H NMR** (500 MHz, DMSO-*d₆*): δ 4.56 (2H, s, CH₂), 5.50 (1H, s (br), OH), 6.92 (1H, d, *J*=8.0 Hz, ArH), 7.17 (1H, dd, *J₁*=8.0 Hz, *J₂*=1.7 Hz, ArH), 7.22 (1H, d, *J*=8.0 Hz, ArH), 7.28 (1H, d, *J*=8.6, ArH), 7.36 (1H, t, *J*=8.0 Hz, ArH), 7.66 (1H, s, ArH), 8.11 (1H, d, *J*=1.7 Hz, ArH), 8.34 (1H, s, NH), 9.74 (1H, s, NH). **MS-ESI⁺** CV 20 V, *m*/*z* (rel. int.): 405.0 [M+H]⁺ (20), 407.0 [M+H]⁺ (20), 427.0 [M+Na] ⁺ (100), 429.0 [M+Na]⁺ (100). **MS-ESI-** CV 20 V, *m*/*z* (rel. int.): 402.8 [M-H]⁻ (100), 404.7 [M-H]⁻(100); **EM** 405.

### Example 13. 1-(3,5-Difluoro-2-hydroxymethylphenyl)-3-(3-trifluoromethoxyphenyl)urea (35)

1-Isocyanato-3-(trifluoromethoxy)benzen (102 mg, 0.5 mmol) was dissolved in THF and 2-(*tert-*butyldimethylsilanyloxymethyl)-3,5-difluoroaniline (0.5 mmol). The product was obtained according to Example 2. White crystal, yield 100 mg (55 %). **¹H NMR** (500 MHz, DMSO-*d₆*): δ 4.51 (2H, s, CH₂), 5.51 (1H, t, *J*=5.2 Hz, OH), 6.83 (1H, t, *J*=9.5 Hz, ArH), 6.93 (1H, d, *J*=7.5 Hz, ArH), 7.29 (1H, d, *J*=7.5 Hz, ArH), 7.37 (1H, t, *J*=8.0 Hz, ArH), 7.64 (1H, s, ArH), 7.73 (1H, d, *J*=11.0 Hz, ArH), 8.58 (1H, s, NH), 9.94 (1H, s, NH). **MS-ESI⁺** CV 15 V, *m*/*z* (rel. int.): 362.9 [M+H]⁺ (100). **MS-ESI⁻** CV 15 V, *m*/*z* (rel. int.): 361.0 [M-H]⁻ (100); **EM** 362.

### Example 14. 1-(3,5-Dichlorophenyl)-3-[2-(2-hydroxyethyl)phenyl]urea (45)

1,3-Dichloro-5-isocyanatobenzene (188 mg, 1.0 mmol) was dissolved in THF and **12a** (251.5 mg, 1.0 mmol) was added with stirring. The RM was stirred at r.t. for 1 h. The crude product was obtained according to Example 2. The pure product was obtained by (CC) (mobile phase-CHCl₃:MeOH, 95:5). White powder, yield 163 mg (50 %). **HPLC:** purity 97.40 %, *R*ₜ = 25.2 min.; **¹H NMR** (500 MHz, DMSO-*d₆*): δ 2.70 (2H, *t, J*=6.7 Hz, CH₂), 3.59 (2H, *t, J*=6.4 Hz, CH₂), 4.90 (1H, *s* (br), OH), 7.00 (1H, *t, J*=7.6 Hz, ArH), 7.09-7.25 (4H, m, ArH), 7.48-7.53 (2H, m, ArH), 7.6 (1H, *d, J*=7.95 Hz, ArH), 8.20 (1H, s, NH), 9.43 (1H, s, NH). **MS-ESI⁺** CV 20 V, *mlz* (rel. int.), with FA: 325.0 [M+H]⁺ (80), 327.0 [M+H]⁺ (55), 347.0 [M+Na]⁺ (100), 349.0 [M+Na]⁺ (70). **MS-ESI⁻** CV 20 V, *m*/*z* (rel. int.), with FA: 323.1 [M-H]⁻ (65), 325.1 [M-H]⁻ (50), 359.0 [M+Cl]⁻(100), 361.0 [M+Cl]⁻ (100); **EM** 324.

### Example 15. 1-[3,5-Dichloro-2-(2-hydroxyethyl)phenyl]-3-(3,5-dichlorophenyl)urea (49)

Potassium *tert*-butoxid (2.0 g, 18.0 mmol) was stirred in dry THF at -40 °C. To this mixture solution of 1,3-dichloro-5-nitrobenzene (1.0 g, 5.2 mmol) and ethyl chloroacetate (0.75 mL, 7.0 mmol) in THF was addeddropwise during 1 h. Then the RM is slowly allowed to warm to 0 °C at which temperature 2.0 M HCl (5 mL) is added. Then the THF was evaporated and the residue was partitioned between water and ethyl acetate. Evaporation of ethyl acetate gave crude ethyl (2,4-dichloro-6-nitrophenyl)-acetate (800 mg, 55 %), which was subsequently reduced with diisobutylaluminum hydride (10 eq) in THF at 0 °C to 2-(2,4-dichloro-6-nitrophenyl)ethanol (670 mg, 99 %). **EM** 235. **MS-APCI⁻** CV 14 V, t=250 °C, *m*/*z* (rel. int.): 235.0 and 237.0 [M-H]⁻ (100 and 60).

2-(2,4-Dichloro-6-nitrophenyl)ethanol (250 mg, 1.06 mmol) was dissolved in etyl acetate (5 mL) and SnCl₂ (570 mg, 3.0 mmol) was added. The RM was stirred at r.t. overnight. After the reaction, an saturated NaHCO₃ (aq) solution was added to the RM until basic pH occured. The RM was centrifuged and the ethyl acetate layer was separated. After evaporation of ethyl acetate, crude 2-(2-amino-4,6-dichlorophenyl)ethanol (200 mg, 98 %) was obtained. **EM** 205. **MS-ESI⁺** CV 15 V, *m*/*z* (rel. int.): 206.0 and 208.0 [M+H]⁺ (100 and 80). **MS-ESI⁻** CV 15 V, *m*/*z* (rel. int.): 203.9 and 205.9 [M-H]⁻ (100 and 70), 240.0 and 242.0 [M+Cl]⁻ (40 and 40). The OH group of the latter substance was protected with TBDMS group to 2-[2-(*tert*-butyldimethyl-silanyloxy)ethyl]-3,5-dichloroaniline which was added (60 mg, 0,18 mmol) to solution of 1,3-dichloro-5-isocyanatobenzene (33 mg, 0,18 mmol) in acetone. This RM was stirred at r.t. for 10 h. The TBDMS-protected product 1-{2-[2-(*tert*-butyldimethylsilanyloxy)ethyl]-3,5-dichlorophenyl}-3-(3,5-dichlorophenyl)urea was isolated by CC (mibile phase - chloroform), and subsequently transformed to hydroxy-derivative according to the example 2. Yield 40 mg (55 %). **¹H NMR** (500 MHz, DMSO-*d₆*): δ 2.90 (2H, t, *J*=6.0 Hz, CH₂), 3.59 (2H, t, *J*=6.5 Hz, CH₂OH), 5.15 (1H, s (br), OH), 7.15 (1H, t, *J*=2.3 Hz, ArH), 7.30 (1H, d, *J*=2.3 Hz, ArH, 7.50 (2H, d, *J*=1.7 Hz, ArH), 7.83 (1H, d, *J*= 1.7 Hz, ArH), 8.46 (1H, s, NH), 9.83 (1H, s, NH). **MS-ESI⁺** CV 20 V, *m*/*z* (rel. int.): 415.0, 417.0 and 419.0 [M+Na]⁺ (80, 100 and 60). **MS-ESI⁻** CV 20 V, *m*/*z* (rel. int.): 391.0, 393.0 and 394.8 [M-H]⁻ (80, 100 and 60), **EM** 392.

### Example 16. 1-(3-Chloro-5-methoxyphenyl)-3-[2-(2-hydroxyethyl)phenyl]urea (55)

1-chloro-3-isocyanato-5-methoxybenzen (64 mg, 0.4 mmol) was dissolved in THF and **12a** (100 mg, 0.4 mmol) was added. The RM was stirred at r.t. for 1 h. The crude product was obtained according to Example 2. The pure product was obtained by crystallization from chloroform. White crystal, yield 100 mg (78 %). **¹H NMR** (500 MHz, DMSO-*d₆*): δ 2.71 (2H, t, *J*=6.7 Hz, CH₂), 3.58 (2H, t, *J*=6.7 Hz, CH₂OH), 3.71 (3H, s, CH₃), 4.87 (1H, s (br), OH), 6.58 (1H, t, *J*=1.8 Hz, ArH), 6.92 (1H, t, *J*=1.8 Hz, ArH), 6.99 (1H, dt, *Jₜ=7.6* Hz, *J_{d}*=1.0 Hz, ArH), 7.13 (1H, dt, *Jₜ*=8.0, *J_{d}*=1.5 Hz, ArH), 7.17 (1H, dd, *J₁*=7.6 Hz, *J₂*=1.2 Hz, ArH), 7.19 (1H, t, *J*=1.5 Hz, ArH), 7.65 (1H, d, *J*=7.3 Hz, ArH), 8.08 (1H, s, NH), 9.26 (1H, s, NH). **MS-ESI⁺** CV 15 V, *m*/*z* (rel. int.): 320.9 [M+H]⁺ (100). **MS-ESI⁻** CV 15 V, *m*/*z* (rel. int.): 318.9 [M-H]⁻ (100), 320.1 [2M-H]⁻ (30); **EM** 320.

### Example 17. 1-[2-(2-Hydroxyethyl)phenyl]-3-(3-trifluoromethoxyphenyl)urea (61)

1-Isocyanato-3-(trifluoromethoxy)benzen (102 mg, 0.5 mmol) was dissolved in THF and **12a** (126 mg, 0.5 mmol) was added. The RM was stirred at r.t. for 1 h. The crude product was obtained according to Example 2. The pure product was obtained by crystallization from hot chloroform. White crystal, yield 73 mg (43 %). **HPLC:** purity 99.6 %, Rt = 24.0 min.; **¹H NMR** (500 MHz, DMSO-d6): δ 2.71 (2H, t, *J*=7.0 Hz, CH2), 3.58 (2H, q, *J*=6.5 Hz, CH2), 4.85 (1H, t, *J*=4.5 Hz, OH), 6.87 (1H, d, *J*=9.0 Hz, ArH), 6.97 (1H, t, *J*=7.5 Hz, ArH), 7.12 (1H, t, *J*=7.5 Hz, ArH), 7.16 (1H, d, *J*=7.5 Hz, ArH), 7.25 (1H, d, *J*=8.0 Hz, ArH), 7.34 (1H, t, *J*=8.0 Hz, ArH), 7.64-7.67 (2H, m, ArH), 8.10 (1H, s, NH), 9.37 (1H, s, NH). **MS-ESI⁺** CV 20 V, *m*/*z* (rel. int.): 340.9 [M+H]⁺ (40), 362.9 [M+Na]⁺ (100). **MS-ESI⁻** CV 20 V, *m*/*z* (rel. int.): 338.9 [M-H]⁻ (90), 374.9 [M+Cl]⁻(100), 376.8 [M+Cl]⁻ (40); **EM** 340.

### Example 18. 1-(3-Chloro-5-trifluoromethoxyphenyl)-3-[2-(2-hydroxyethyl)phenyl]urea (64)

1-chlor-3-isocyanato-5-(trifluoromethoxy)benzen (118 mg, 0.5 mmol) was dissolved in DCM and **12a** (126 mg, 0.5 mmol) was added with stirring. The RM was stirred at 50 °C for 5 h. The crude product was obtained according to Example 2. The pure product was obtained by CC (mobile phase - 5% MeOH in chloroform). White crystal, yield 64 mg (34 %). **HPLC:** purity 98.6 %, *R*ₜ = 25.2 min.; **¹H NMR** (500 MHz, DMSO-*d₆*): δ 2.71 (2H, t, *J*=6.9 Hz, CH₂), 3.60 (2H, q, *J*=4.6 Hz, CH₂), 4.92 (1H, t, *J*=4.6 Hz, OH), 7.01 (1H, t, *J*=7.5 Hz, ArH), 7.05 (1H, s, ArH), 7.15 (1H, t, *J*=7.5 Hz, ArH), 7.18 (1H, d, *J*=8.0 Hz, ArH), 7.49 (1H, s, ArH), 7.54 (1H, t, *J*=1.7 Hz, ArH), 7.61 (1H, d, *J*=7.5 Hz, ArH), 8.24 (1H, s, NH), 9.56 (1H, s, NH). **MS-ESI⁺** CV 20 V, *m*/*z* (rel. int.): 375.1 and 377.1 [M+H]⁺ (70 and 25), 397.1 and 399.1 [M+Na]⁺ (100 and 35). **MS-ESI⁻** CV 20 V, *m*/*z* (rel. int.): 373.0 and 375.0 [M-H]⁻ (80 and 30), 409.0 and 411.0 [M+Cl]⁻ (100 and 60); **EM** 374.

### Example 19. 1-(3-Bromo-5-trifluoromethoxyphenyl)-3-[2-(2-hydroxyethyl)phenyl]urea (65)

1-Bromo-3-isocyanato-5-(trifluoromethoxy)benzen (282 mg, 1.0 mmol) was dissolved in DCM and **12a** (252 mg, 1.0 mmol) was added. The RM was stirred at r.t. for 5 h. The product was obtained according to Example 2. White crystals, yield 348 mg (83 %). **HPLC:** purity 99.3 %, *R*ₜ = 25.5 min.; **¹H NMR** (500 MHz, DMSO-*d₆*): δ 2.71 (2H, t, *J*=6.3 Hz, CH₂), 3.60 (2H, q, *J*=4.6 Hz, CH₂), 4.92 (1H, t, *J*=4.0 Hz, OH), 7.01 (1H, t, *J*=7.5 Hz, ArH), 7.11-7.21 (3H, m, ArH), 7.52 (1H, s, ArH), 7.61 (1H, d, *J*=8.0 Hz, ArH), 7.68 (1H, s, ArH), 8.23 (1H, s, NH), 9.56 (1H, s, NH). **MS-ESI⁺** CV 20 V, *m*/*z* (rel. int.): 440.4 and 442.2 [M+Na]⁺ (100, 90). **MS-ESI⁻** CV 20 V, *m*/*z* (rel. int.): 416.5 and 418.6 [M-H]⁻ (97, 100), 452.5 and 454.5 [M+Cl]⁻ (30, 55); **EM** 419.

### Example 20. 1-[3-(2-Hydroxyethylamino)-5-trifluoromethoxyphenyl]-3-[2-(2-hydroxyethyl)phenyl]urea (66)

1-(3-Bromo-5-(trifluoromethoxy)phenyl)-3-[2-(2-hydroxyethyl)phenyl]urea (**65,** 134 mg, 0.32 mmol) was dissolved in aminoethan-1-ol (0.5 mL). Into this RM CuCl (3.0 mg, 0.03 mmol) and KOH (33 mg, 0.6 mmol) was added and the RM was stirred at 90 °C for 4 h. After reaction, ethanol (1 mL) was added and solvents were evaporated. The residue was partitioned between water and diethyl ather. Pure product was obtained by CC (mobile phase - 7% methanol in chloroform). White powder, yield 28 mg (22 %). **HPLC:** purity 97.7 %, *R*ₜ = 22.4 min.; **¹H NMR** (500 MHz, DMSO-*d₆*): δ 2.70 (2H, t, *J*=7.0 Hz, CH₂CH₂OH), 3.03 (2H, q, *J*=5.5 Hz, CH₂), 3.50 (2H, q, *J*=5.5 Hz, CH₂), 3.57 (2H, q, *J*=4.5 Hz, CH₂), 4.70 (1H, t, *J*=5.0 Hz, OH), 7.83 (1H, t, *J*=4.5 Hz, OH), 5.99 (1H, t, *J*=5.0 Hz, NHCH₂CH₂OH), 6.09 (1H, s, ArH), 6.52 (1H, s, ArH), 6.76 (1H, s, ArH), 6.95(1H, t, *J*=7.5 Hz, ArH), 7.11 (1H, t, *J*=8.0 Hz, ArH), 7.15 (1H, d, *J*=7.5 Hz, ArH), 7.69 (1H, d, *J*=8.0 Hz, ArH), 7.99 (1H, s, NH), 9.12 (1H, s, NH). **MS-ESI⁺** CV 20 V, *m*/*z* (rel. int.): 421.9 [M+Na]⁺ (100), 821.1 [2M+Na]⁺(60). **MS-ESI⁻** CV 20 V, *m*/*z* (rel. int.): 398.0 [M-H]⁻ (100); **EM** 399.

### Example 21. 1-[2-(2-Hydroxyethyl)phenyl]-3-(3-trifluoromethylsulphanylphenyl)urea (68)

1-Isocyanato-3-(trifluoromethylsulphanyl)benzen (110 mg, 0.5 mmol) was dissolved in DCM and **12a** (125 mg, 0.5 mmol) was added. The RM was stirred at r.t for 2 h. The product was obtained according to Example 2 and recrystallized from diethyl ether. White crystals, yield 124 mg (70 %). **HPLC:** purity 98.6 %, At = 24.5 min.; **¹H NMR** (500 MHz, DMSO-*d₆*): δ 2.71 (2H, t, J=6.9 Hz, CH₂), 3.58 (2H, q, J=4.6 Hz, CH₂OH), 4.85 (1H, t, J=4.6 Hz, OH), 6.97 (1H, t, J=7.7 Hz, ArH), 7.12 (1H, t, J=7.5 Hz, ArH), 7.15 (1H, d, J=7.5 Hz, ArH), 7.23 (1H, d, J=7.5 Hz, ArH), 7.38 (1H, t, J=8.0 Hz, ArH), 7.52 (1H, d, J=8.0 Hz, ArH), 7.66 (1H, d, J=8.0 Hz, ArH), 7.93 (1H, s, ArH), 8.11 (1H, s, NH), 9.38 (1H, s, NH). **MS-ESI⁺** CV 20 V, *m*/*z* (rel. int.): 356.9 [M+H]⁺(100), 378.8 [M+Na]⁺ (60), 734.8 [2M+Na]⁺ (60). **MS-ESI⁻** CV 20 V, *m*/*z* (rel. int.): 354.8 [M-H]⁻(100); **EM** 356.

Additional compounds listed in Table 1. were prepared following the procedures in Examples 1-21.

**Table 1. Overview of substituents in positions R1-R4 for the synthesised compounds, their MS-ESI [M+H]⁺ values and IC₅₀ values in units of micromoles with enzyme ZmCKX1. If the substituent is not specified, hydrogen (H) is in the given position. Explanations: HM-hydroxymethyl group, HE - hydroxyethyl group, CP-chloropyridyl, P - pyridyl, D - di, F - fluorine, Cl (C) - chlorine, Br - bromine, MeO - methoxy group, MeS - methylthio group, TFM-trifluoromethoxy group, TFS - trifluoromethylsulphanyl group, AEA - aminoethylamino group, AEH - aminoethylhydroxy group. The number indicates the position of the substituent on the phenyl in relation to the urea bridge; a dash separates the two aromatic rings. Abbreviations do not contain the words phenyl and urea (except for the standard - 3,5DCl-Ph-U). Abbreviations begin with a pyridyl ring or later, where X = C with ring, which does not carry an alcohol group.**

| Comp. number | Comp. abbreviation | n = | R1 | R2 | R3 | R4 | MS-ESI [M+H] | IC₅₀ (µM) ZmCKX1 |
|---|---|---|---|---|---|---|---|---|
| | | Compounds wherein X=N | | | | | | |
| | CPPU (standard) | 0 | Cl | | | | | 20.5 |
| | DCPPU (standard) | 0 | Cl | Cl | | | | 4.5 |
| **1** | FP-2HM | 1 | F | | | | 262.1 | 2.5 |
| **2** | FP-2HM,5Cl | 1 | F | | | Cl | 296.0 | 0.27 |
| **3** | DFP-2HM | 1 | F | F | | | 279.9 | 0.82 |
| **4** | CP-2HM | 1 | Cl | | | | 278.2 | 1.6 |
| **5** | DCP-2HM | 1 | Cl | Cl | | | 312.1 | 0.70 |
| **6** | DCP-2HM,5Cl | 1 | Cl | Cl | | Cl | 346.1 | 0.10 |
| **7** | MeOP-2HM | 1 | OCH₃ | | | | 274.0 | 1.1 |
| **8** | MeOP-2HM,5Br | 1 | OCH₃ | | | Br | 352.9 | 0.13 |
| **9** | FP-2HE | 2 | F | | | | 276.1 | 1.6 |
| **10** | DFP-2HE | 2 | F | F | | | 294.1 | 0.72 |
| **11** | DFP-2HE,5Cl | 2 | F | F | | Cl | 328.1 | 0.095 |
| **12** | CP-2HE | 2 | Cl | | | | 292.0 | 1.3 |
| **13** | DCP-2HE | 2 | Cl | Cl | | | 326.2 | 0.43 |
| **14** | DCP-2HE,5F | 2 | Cl | Cl | | F | 344.1 | 0.056 |
| **15** | MeOP-2HE,5F | 2 | OCH₃ | | | F | 306.0 | 0.15 |
| **16** | MeOP-2HE,3,5DC | 2 | OCH₃ | | Cl | Cl | 356.0 | 0.081 |
| | | Compounds wherein X=C | | | | | | |
| | 3,5DCl-Ph-U (standard) | 0 | | | | | | 0.84 |
| **17** | 3,5DF-2HM | 1 | F | F | | | 279.0 | 0.65 |
| **18** | 3,5DC1-2HM | 1 | Cl | Cl | | | 311.1 | 0.40 |
| **19** | 3,5DBr-2HM | 1 | Br | Br | | | 401.0 | 0.33 |
| **20** | 3,5DC-2HM,3Cl | 1 | Cl | | Cl | Cl | 344.8 | 0.27 |
| **21** | TCl,2HM | 1 | Cl | Cl | Cl | Cl | 379.1 | 0.02 |
| **22** | 3MeO,5Br-2HM | 1 | OCH₃ | Br | | | 351.8 | 0.26 |
| **23** | 3MeO,5AEH-2HM | 1 | OCH₃ | AEH | | | 332.0 | 0.20 |
| **24** | 3MeO,5AEA-2HM | 1 | OCH₃ | AEA | | | 331.1 | 0.18 |
| **25** | 3MeO,5AEA-2HM,5Cl | 1 | OCH₃ | AEA | | Cl | 365.0 | 0.024 |
| **26** | 3MeS-2HM | 1 | SCH₃ | | | | 288.9 | 1.5 |
| **27** | 3MeS-2HM,5Br | 1 | SCH₃ | | | Br | 367.9 | 0.17 |
| **28** | 3TFM-2HM | 1 | OCF₃ | | | | 327.0 | 1.3 |
| **29** | 3TFM-2HM,3F | 1 | OCF₃ | | F | | 344.8 | 0.36 |
| **30** | 3TFM-2HM,3Cl | 1 | OCF₃ | | Cl | | 361.1 | 0.30 |
| **31** | 3TFM-2HM,3Br | 1 | OCF₃ | | Br | | 406.1 | 0.37 |
| **32** | 3TFM-2HM,5F | 1 | OCF₃ | | | F | 344.9 | 0.11 |
| **33** | 3TFM-2HM,5Cl | 1 | OCF₃ | | | Cl | 361.0 | 0.060 |
| **34** | 3TFM-2HM,5Br | 1 | OCF₃ | | | Br | 405.9 | 0.10 |
| **35** | 3TFM-2HM,3,5DF | 1 | OCF₃ | | F | F | 363.1 | 0.085 |
| **36** | 3TFM-2HM,3,5DCl | 1 | OCF₃ | | Cl | Cl | 395.0 | 0.032 |
| **37** | 3TFM-2HM,3,5DBr | 1 | OCF₃ | | Br | Br | 485.0 | 0.051 |
| **38** | 3TFM,5AEH-2HM | 1 | OCF₃ | AEH | | | 386.1 | 0.10 |
| **39** | 3TFM,5AEA-2HM | 1 | OCF₃ | AEA | | | 385.2 | 0.095 |
| **40** | 3TFM,5AEA-2HM, 5Cl | 1 | OCF₃ | AEA | | Cl | 419.0 | 0.010 |
| **41** | 3TFS-2HM | 1 | SCF₃ | | | | 343.1 | 0.14 |
| **42** | 3TFS-2HM,5F | 1 | SCF₃ | | | F | 360.9 | 0.080 |
| **43** | 3TFS-2HM,5Cl | 1 | SCF₃ | | | Cl | 376.9 | 0.020 |
| **44** | 3,5DF-2HE | 2 | F | F | | | 293.0 | 0.36 |
| **45** | 3,5DC-2HE | 2 | Cl | Cl | | | 325.0 | 0.18 |
| **46** | 3,5DBr-2HE | 2 | Br | Br | | | 414.9 | 0.14 |
| **47** | 3Br,5F-2HE | 2 | Br | F | | | 354.1 | 0.17 |
| **48** | 3Cl,5F-2HE | 2 | Cl | F | | | 309.0 | 0.21 |
| **49** | TCl,2HE | 2 | Cl | Cl | Cl | Cl | 392.9 | 0.010 |
| **50** | 3MeO-2HE | 2 | OCH₃ | | | | 287.1 | 0.27 |
| **51** | 3MeO-2HE,5F | 2 | OCH₃ | | | F | 305.1 | 0.080 |
| **52** | 3MeO-2HE,5Cl | 2 | OCH₃ | | | Cl | 321.1 | 0.066 |
| **53** | 3MeO-2HE,5Br | 2 | OCH₃ | | | Br | 366.0 | 0.074 |
| **54** | 3MeO,5F-2HE | 2 | OCH₃ | F | | | 305.0 | 0.28 |
| **55** | 3MeO,5Cl-2HE | 2 | OCH₃ | Cl | | | 321.1 | 0.18 |
| **56** | 3MeO,5Br-2HE | 2 | OCH₃ | Br | | | 366.1 | 0.14 |
| **57** | 3MeO,5AEH-2HE | 2 | OCH₃ | AEH | | | 346.0 | 0.16 |
| **58** | 3MeO,5AEA-2HE | 2 | OCH₃ | AEA | | | 345.2 | 0.13 |
| **59** | 3MeO,5AEA-2HE,5Cl | 2 | OCH₃ | AEA | | Cl | 378.9 | 0.008 |
| **60** | 3MeS-2HE,5Cl | 2 | SCH3 | | | Cl | 337.0 | 0.035 |
| **61** | 3TFM-2HE | 2 | OCF₃ | | | | 340.9 | 0.18 |
| **62** | 3TFM-2HE,5Cl | 2 | OCF₃ | | | Cl | 374.9 | 0.021 |
| **63** | 3TFM-2HE, 5F | 2 | OCF₃ | | | F | 359.0 | 0.036 |
| **64** | 3TFM,5Cl-2HE | 2 | OCF₃ | Cl | | | 375.0 | 0.058 |
| **65** | 3TFM,5Br-2HE | 2 | OCF₃ | Br | | | 420.1 | 0.035 |
| **66** | 3TFM,5AEH-2HE | 2 | OCF₃ | AEH | | | 399.9 | 0.029 |
| **67** | 3TFM,5AEA-2HE | 2 | OCF₃ | AEA | | | 399.0 | 0.022 |
| **68** | 3TFS-2HE | 2 | SCF₃ | | | | 357.0 | 0.074 |

### Example 22. Compounds inhibit the activity of the maize enzyme cytokinin oxidase/dehydrogenase 1 (ZmCKX1)

The *IC*₅₀ (concentration of a substance that causes 50% inhibition of the enzyme activity) was measured by PMS/MTT assay [phenazine methosulphate/3-(4,5-dimethylthiazol-2-yl)-2,5-diphenyltetrazolium bromide]. The assay was performed in 96-well microtiter plates. Each well contained a reaction mixture with a final concentration of: 0.1 M KH₂PO₄, pH 7.4, 1 mM MTT, 0.2 mM PMS, tested substance in a concentration range of 0.1 nM - 1 µM and 10 µM N⁶-isopentenyladenine (iP) as a substrate. To this reaction mixture 100 µL of medium containing the active enzyme ZmCKX1 was added. The enzyme was produced by the yeast *Yarrowia lipolytica* and isolated according to the protocol (Kopečný D. et al., 2005, Biochemistry 87:1011-1022). The plates were incubated for 30 min at 37 °C and the enzymatic reaction was stopped by the addition of 25 µL of 35% acetic acid. Then, the absorbance at 578 nm was measured on a spectrophotometer, and the absorbance of the sample without the substrate was substracted as a reference value. The *IC*₅₀ value was determined from the concentration-dependent curves in GraphPad Prism software. The values shown in Table 1 are the average of at least two measurements each performed in three repetitions. All of the mentioned CPPU or DCPPU derivatives have significantly lower *IC*₅₀ values than their parent molecules CPPU and DCPPU, which are not substituted on the phenyl ring. This also applies to 3,5-DCl-Ph-U derivatives.

### Example 23. The compounds show anti-senescence activity in a wheat leaf senescence assay performed in the dark

Seeds of winter wheat (*Triticum aestivum* cv. Hereward) was washed under running water for 4 hours and then sown in vermiculite saturated with Knop's nutrient solution. The trays with seeds were placed in an air-conditioned growth chamber with a 16/8 hour light regime (light intensity 50 µmol.m⁻².s⁻¹) and a temperature of 15 °C. After 7 days, the seedlings had developed the first flag leaf and the second leaf began to grow. Top sections approximately 4 cm long were excised from the first leaves of 4 plants and their combined weight was adjusted to exactly 100 mg. The basal ends of these 4 leaf segments were placed in the wells of microtiter polystyrene plates containing solution (150 µL) of the tested derivative. The plates were placed in a plastic box with filter paper, which was saturated with water (to maintain the maximum humidity). After 96 hours of incubation in the dark at 25 °C, the leaf segments were removed and the chlorophyll was extracted to 5 mL of 80% ethanol by heating the leaf segments in ethanol at 80 °C for 40 min. The sample volume was then adjusted to 5 mL by adding 80% ethanol. The absorbance of the extracts was measured at 665 nm. Chlorophyll extracts from leaf segments incubated in deionized water (so-called yellow control) were also measured as controls. Freshly cut leaves (100 mg) were used as a green control, from which chlorophyll was extracted and measured in the same manner. The calculated values are the average of 5 replicates and the whole experiment was repeated two times. The tested compounds were dissolved in dimethyl sulfoxide (DMSO) to a concentration 10⁻² M. This stock solution was further diluted with water to a concentration of 10 micromolar. At this concentration, the substances were tested. The final concentration of DMSO in the water did not exceed 0.2 %.

All tested compounds of the general formula (Ia) show a positive effect on retention of chllorophyl in leaf segments, which were dettached from plant and placed in darkness. All newly prepared and tested diphenyl urea derivatives of formula (Ia) maintained chlorophyll levels at least above 23 % compared to the yellow control (Table 2).

**Table 2. The effect of new compounds on retention of chllorophyl in decapitated segments of wheat leaves (Triticum aestivum cv. Hereward) in the dark.**

| Compound number | Compound abbreviation | (% of chlorophyll above control) |
|---|---|---|
| yellow control (0,2% DMSO) | | 0% |
| standard | CPPU | 12 % |
| **2** | FP-2HM,5Cl | 23 % |
| **6** | DCP-2HM,5Cl | 29 % |
| **8** | MeOP-2HM,5Br | 25 % |
| **11** | DFP-2HE,5Cl | 31 % |
| **16** | MeOP-2HE,3,5DC | 35 % |
| **22** | 3MeO,5Br-2HM | 25 % |
| **25** | 3MeO,5AEA-2HM,5Cl | 45 % |
| **27** | 3MeS-2HM,5Br | 26 % |
| **34** | 3TFM-2HM,5Br | 32 % |
| **40** | 3TFM,5AEA-2HM, 5Cl | 39 % |
| **42** | 3TFS-2HM,5F | 25 % |
| **51** | 3MeO-2HE,5F | 30 % |
| **52** | 3MeO-2HE,5Cl | 37 % |
| **53** | 3MeO-2HE,5Br | 35 % |
| **59** | 3MeO,5AEA-2HE,5Cl | 48 % |
| **60** | 3MeS-2HE,5Cl | 36 % |
| **61** | 3TFM-2HE | 24 % |
| **62** | 3TFM-2HE,5Cl | 39 % |
| **65** | 3TFM,5Br-2HE | 30 % |
| **66** | 3TFM,5AEH-2HE | 36 % |
| **68** | 3TFS-2HE | 34 % |

### Example 24. Compound 61 reduces the effects of abiotic stress on the development of Arabidopsis seedlings

Compound **61** was tested for its ability to alleviate the effects of abiotic stress, represented by application of 100 mM NaCl (saline stress) or 100 mM mannitol (osmotic stress), respectively. The assay was performed as described in (Ugena et al., 2018, Front Plant Sci. 9, 1327. doi: 10.3389/fpls.2018.01327). Arabidopsis thaliana (ecotype Col-0) seeds were germinated on full MS growth medium for three days without presence of any treatment. After that they were transferred to the same medium containing 10 nM concertation of compound **61** in the absence or presence of the abiotic stress factor. Dimethyl sulfoxide (DMSO) was used as a solvent control (mock) in all variants. The dynamics of the shoot biomass formation and the relative growth rate were evaluated by recording the green area twice a day during the 6 days of growth after the seedling transfer. The abiotic stress conditions caused severe penalty in both traits evaluated. The salt and mannitol stress reduced the shoot yield by 63 % and 42 %, respectively, whereas presence of the compound **61** turned back the penalty by almost 20 % (Table 3 and Fig. 1). The same was observed in the case of the average relative growth rate that was lowered to 51 % and 62 % by the two stressors, whereas the simultaneous presence of the compound **61** reduced the effect of the stressors by app. 11 % (Table 3).

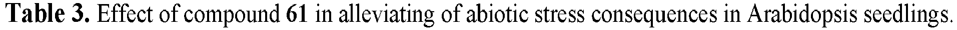

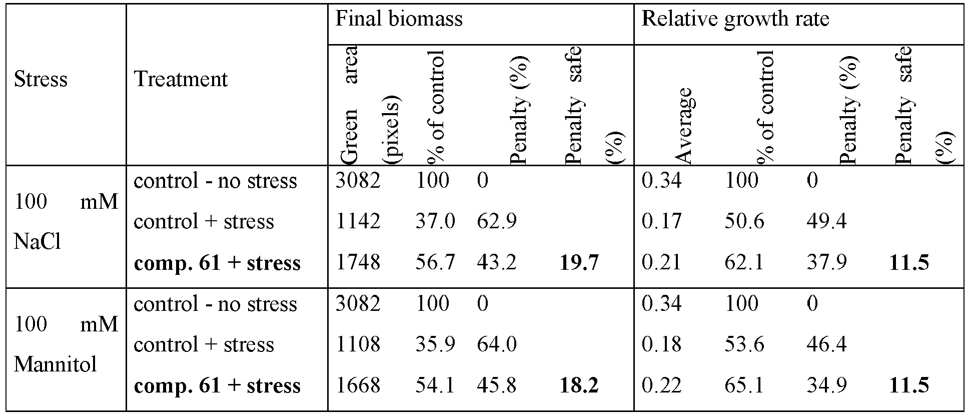

### Example 25. Foliar application of compound 61 increases the seed yield in Arabidopsis

It has previously been shown that a reduction of CKX activity in Arabidopsis has led to an increase in the number of flowers/pods and an increase in the number of seeds in each silique. The result was a 55% increase in total seed yield compared to control plants (Bartrina et al., 2011, Plant Cell 23, 69-80).

In our experiment, young *Arabidopsis thaliana* plants were grown in small pots in soil and they were sprayed with a 0.1 µM compound **61** (aq solution) in phase of 4 true leaves (about 14-20 days after sowing). Treated plants (n = 25) and control plants (n = 25) were further grown under optimal conditions until their natural death. Subsequently, seeds were harvested from all plants and the yield was evaluated. While control plants had an average of 44 (± 8) seeds per silique and a total seed yield per plant was 130 (± 16) mg, treated plants had an average of 52 (± 4) seeds per silique and a total seed yield per plant was 155 (± 19) mg. This means a 19 % increase in total seed yield.

This whole experiment was repeated with a similar result: control plants had an average of 40 (± 6) seeds per silique and a total seed yield per plant was 112 (± 13) mg. Treated plants had an average of 49 (± 6) seeds per one silique and the total seed yield per plant was 138 (± 15) mg. This means an increase in total seed yield of 23 %.

### Example 26. Foliar application with compound 61 prolongs the lifespan of Arabidopsis thaliana under optimal conditions

Young *Arabidopsis thaliana* plants grown in small pots in a soil were sprayed with a 0.1 µM aqueous solution of substance 61 in phase of 4 true leaves (within 14-20 days after sowing).

Treated plants (n = 25) and control plants (n = 25) were further grown under optimal conditions until their natural death as in Example 25. The treated plants showed an average of 10 days longer viability. The treated plants remained more green for a longer period of time (delayed senescence), which allowed them to accumulate more products of photosynthesis, which in turn may have contributed to a higher yield of these plants.

### Example 27. The application of compound 61 increases the yield of winter rapeseed in field conditions

In 2017, the effect of foliar application of compound **61** (5 µM concentration in a volume of 300 L per hectare) on seed yield of winter rapeseed in field conditions was determined. The field trials were performed according to "good agricultural practice". Substance **61** was applied to plants in two different growth phases - BBCH 30 and BBCH 33-35. In the first case, the yield increased to 105.7 % compared to the control, and in the second case, the seed yield increased to 107.5 % compared to the control (100 %). Furthermore, the seeds of the treated plants were found to be slightly heavier, the weight of one thousand seeds in the control was 5.51 g. The weight of one thousand seeds in the treated plants was 5.7 g (BBCH 30) and 5.8 g (BBCH 33-35). This phenomenon contributed to the increase in total seed yield.

### Example 28. Compounds 64 and 66 delay the senescence of wheat plants under saline stress

Salinity is one of the major problems that negatively affects soil fertility and limits plant growth and production (Richards, L. A. (Ed.) 1954. USDA Agriculture Handbook 60, Washington D. C.). Under saline stress, physiological drought can occur, excess salt in plants disrupts cellular functions and impairs physiological processes such as photosynthesis and respiration (Staples, RC, Toenniessen GH (Eds.), Salinity Tolerance in Plants. John Wiley and Sons, New York: 67-76, Marschner H., 1995. Mineral Nutrition of Higher Plants), which together lead to stress-induced senescence of plant organs, and can result in plant death.

To investigate the effects of compounds **64** and **66** on salt-induced senescence, seeds of winter wheat (*Triticum aestivum* cv. Hereward) were washed under running water for 4 hours and then sown on perlite soaked with water (control) or 100 nM aqueous solution of compounds **64** or **66.** Trays with seeds were placed in a growth chamber at 25 °C for 16/8 h light/dark at an intensity of 50 µmol.m⁻².s⁻¹. Seven days after planting, the plants were treated with 75 mM NaCl solution. The plants were allowed to grow and the plant damage was assessed 30 days after planting.

The use of compound **64** or **66** has reduced the damage and delayed the senescence of wheat plants under salinity stress conditions. While untreated plants have the first leaves dead and the second leaves yellow, plants treated with CKX inhibitors have the first leaves yellowing and the second leaves green. Analysis of total chlorophyll content in whole plants showed that untreated salinity-stressed plants have on average 27 % less chlorophyll than plants stressed but treated with substances **64** or **66.** These data clearly show that CKX inhibitors significantly delay stress-induced senescence of wheat plants. This *in vivo* experiment is evidence that CKX inhibitors prolong the photosynthetic activity of wheat plants that have been exposed to environmental stress.

### Example 29. Formulations

The active ingredient in the formulations may be any compound of formula (I) or (Ia).

| A1. Emulsifiable concentrates | a) | b) | c) | d) |
|---|---|---|---|---|
| active ingredient mixture | 5% | 10% | 25 % | 50% |
| calcium dodecylbenzenesulfonate | 6 % | 8 % | 6 % | 8 % |
| castor oil polyglycol ether (36 mol of ethylene oxide) | 4 % | - | 4 % | 4 % |
| octylphenol polyglycol ether (7-8 mol of ethylene oxide) | 2 % | - | 2 % | - |
| cyclohexanone | - | - | 10% | 20% |
| arom. hydrocarbon mixture C₉-C₁₂ | 83 % | 82 % | 53 % | 18 % |

Emulsions of any desired concentration can be obtained from such concentrates by dilution with water.

| A2. Solutions | a) | b) | c) | d) |
|---|---|---|---|---|
| active ingredient mixture | 5% | 10% | 50% | 90% |
| 1-methoxy-3-(3-methoxy-propoxy)-propane | - | 20 % | 20 % | - |
| polyethylene glycol MW 400 | 20 % | 10 % | - | - |
| N-methyl-2-pyrrolidone | - | - | 30% | 10% |
| arom. hydrocarbon mixture C₉-C₁₂ | 75 % | 60 % | - | - |

The solutions are suitable for use in the form of microdrops.

| A3. Wettable powders | a) | b) | c) | d) |
|---|---|---|---|---|
| active ingredient mixture | 5 % | 25 % | 50 % | 80 % |
| sodium lignosulfonate | 4 % | - | 3 % | - |
| sodium lauryl sulfate | 2 % | 3 % | - | 4 % |
| sodium diisobutylnaphthalene-sulfonate | - | 6 % | 5 % | 6 % |
| octylphenol polyglycol ether (7-8 mol of ethylene oxide) | 1 % | 2 % | - | - |
| highly dispersed silicic acid | 1 % | 3 % | 5% | 10% |
| kaolin | 87 % | 61 % | 37 % | - |

The active ingredient is mixed thoroughly with the adjuvants and the mixture is thoroughly ground in a suitable mill, affording wettable powders which can be diluted with water to give suspensions of any desired concentration.

| A4. Coated granules | a) | b) | c) |
|---|---|---|---|
| active ingredient mixture | 0.1 % | 5 % | 15 % |
| highly dispersed silicic acid | 0.9 % | 2 % | 2 % |
| inorganic carrier (.AE 0.1 -1 mm) e.g. CaCO₃ or SiO₂ | 99.0 % | 93 % | 83 % |

The active ingredient is dissolved in methylene chloroide and applied to the carrier by spraying, and the solvent is then evaporated off in vacuo.

| A5. Coated granules | a) | b) | c) |
|---|---|---|---|
| active ingredient mixture | 0.1 % | 5% | 15 % |
| polyethylene glycol MW 200 | 1.0 % | 2 % | 3 % |
| highly dispersed silicic acid | 0.9 % | 1 % | 2 % |
| inorganic carrier (AE 0.1 -1 mm) e.g. CaCO₃ or SiO₂ | 98.0 % | 92 % | 80 % |

The finely ground active ingredient is uniformly applied, in a mixer, to the carrier moistened with polyethylene glycol. Non-dusty coated granules are obtained in this manner.

| A6. Extruder granules | a) | b) | c) | d) |
|---|---|---|---|---|
| active ingredient mixture | 0.1 % | 3 % | 5 % | 15 % |
| sodium lignosulfonate | 1.5 % | 2 % | 3 % | 4 % |
| carboxymethylcellulose | 1.4 % | 2 % | 2 % | 2 % |
| kaolin | 97 % | 93 % | 90 % | 79 % |

The active ingredient is mixed and ground with the adjuvants, and the mixture is moistened with water. The mixture is extruded and then dried in a stream of air.

| A7. Dusts | a) | b) | c) |
|---|---|---|---|
| active ingredient mixture | 0.1 % | 1 % | 5 % |
| talcum | 39.9 % | 49 % | 35 % |
| kaolin | 60 % | 50 % | 60 % |

Ready-to-use dusts are obtained by mixing the active ingredient with the carriers and grinding the mixture in a suitable mill.

| A8. Suspension concentrates | a) | b) | c) | d) |
|---|---|---|---|---|
| active ingredient mixture | 3 % | 10 % | 25 % | 50 % |
| ethylene glycol | 5 % | 5 % | 5 % | 5 % |
| nonylphenol polyglycol ether (15 mol of ethylene oxide) | 1 % | 2 % | - | - |
| sodium lignosulfonate | 3 % | 3 % | 4 % | 5 % |
| carboxymethylcellulose | 1 % | 1 % | 1 % | 1 % |
| 37% aqueous formaldehyde solution | 0.2 % | 0.2 % | 0.2 % | 0.2 % |
| silicone oil emulsion | 0.8 % | 0.8 % | 0.8 % | 0.8 % |
| water | 86 % | 78 % | 64 % | 38 % |

The finely ground active ingredient is intimately mixed with the adjutants, giving a suspension concentrate from which suspensions of any desired concentration can be obtained by dilution with water.

## Claims

1. A compound of the general formula (I), 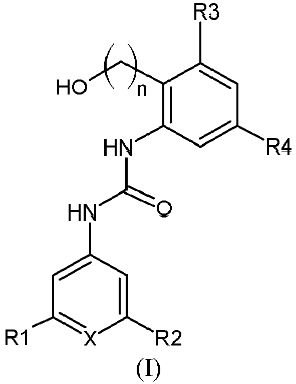 wherein
n is 1 or 2;
X is nitrogen or carbon;
R1 is selected from the group consisting of halogen, methoxy group, trifluoromethoxy group, methylsulphanyl group and trifluoromethylsulphanyl group;
R2 is selected from the group consisting of hydrogen, halogen, amino group, C₁-C₅ alkyl, C₂-C₅ alkenyl, C₂-C₅ alkynyl, and C₁-C₅ alkylamino group, wherein C₁-C₅ alkyl, C₂-C₅ alkenyl, C₂-C₅ alkynyl and C₁-C₅ alkylamino group is optionally substituted with hydroxy and/or amino group;
R3 and R4 are independently hydrogen or halogen;
with the proviso that when X is nitrogen, then R2 is hydrogen or halogen;
and with the proviso that 1-(2,6-dichloropyridin-4-yl)-3-[2-(2-hydroxyethyl)phenyl]urea, 1-[2-(2-hydroxyethyl)phenyl]-3-(3-(trifluoromethoxy)phenyl)urea, 1-[2-(2-hydroxyethyl)phenyl]-3-(3-methoxyphenyl)urea, 1-(3,5-dichlorophenyl)-3-[2-(2-hydroxyethyl)phenyl]urea, 1-(3,5-difluorophenyl)-3-[2-(2-hydroxyethyl)phenyl]urea, 1-(3-fluorophenyl)-3-[2-(2-hydroxyethyl)phenyl]urea, 1-(3-chlorophenyl)-3-[2-(2-hydroxyethyl)phenyl]urea, 1-(2-hydroxymethylphenyl)-3 -(3 -methoxyphenyl)urea, 1-(3,5-dichlorophenyl)-3-(2-hydroxymethylphenyl)urea, 1-(3-fluorophenyl)-3-(2-hydroxymethylphenyl)urea, and 1-(3-chlorophenyl)-3-(2-hydroxymethylphenyl)urea are excluded.

2. The compound according to claim 1, wherein R1 is selected from the group consisting of fluorine, chlorine, bromine, methoxy group, trifluoromethoxy group, methylsulphanyl group and trifluoromethylsulphanyl group.

3. The compound according to claim 1 or 2, wherein R2 is selected from the group consisting of hydrogen, fluorine, chlorine, bromine, amino group, 2-aminoethylamino group and 2-hydroxyethylamino group.

4. The compound according to claim 1, 2 or 3, wherein R3 and R4 are independently selected from the group consisting of hydrogen, fluorine, chlorine and bromine.

5. The compound according to any one of the preceding claims 1 to 4, selected from the group consisting of: 1-(2,6-dichloropyridin-4-yl)-3-(2-hydroxymethylphenyl)urea, 1-(5-chloro-2-hydroxymethylphenyl)-3-(2,6-dichloropyridin-4-yl)urea, 1-(2-methoxypyridin-4-yl)-3-(2-hydroxymethylphenyl)urea, 1-(5-chloro-2-hydroxymethylphenyl)-3-(2-methoxypyridin-4-yl)urea, 1-(2-hydroxymethylphenyl)-3-(3-trifluoromethoxyphenyl)urea, 1-(5-chloro-2-hydroxymethylphenyl)-3-(3-trifluoromethoxyphenyl)urea, 1-(3-chloro-5-trifluoromethoxyphenyl)-3-(2-hydroxymethylphenyl)urea, 1-(3-bromo-5-trifluoromethoxyphenyl)-3-(2-hydroxymethylphenyl)urea, 1-(3-chloro-5-methoxyphenyl)-3-(2-hydroxymethylphenyl)urea, 1-(3 -bromo-5 -methoxyphenyl)-3 -(2-hydroxymethylphenyl)urea, 1-(3-bromo-5-chlorophenyl)-3-(2-hydroxymethylphenyl)urea, 1-[2-(2-hydroxyethyl)phenyl]-3-(3-trifluoromethoxyphenyl)urea, 1-(3-chloro-5-methoxyphenyl)-3-[2-(2-hydroxyethyl)phenyl]urea, 1-(3-bromo-5-methoxyphenyl)-3-[2-(2-hydroxyethyl)phenyl]urea.

6. Use of a compound of the general formula (Ia) 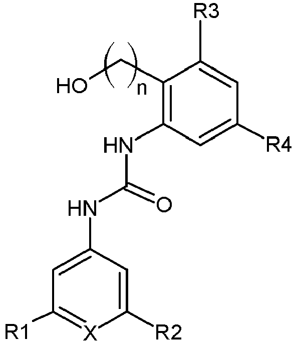 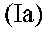 wherein
n is 1 or 2;
X is nitrogen or carbon;
R1 is selected from the group consisting of hydrogen, halogen, methoxy group, trifluoromethoxy group, methylsulphanyl group or trifluoromethylsulphanyl group;
R2 is selected from the group consisting of hydrogen, halogen, amino group, C₁-C₅ alkyl, C₂-C₅ alkenyl, C₂-C₅ alkynyl, and C₁-C₅ alkylamino group, wherein C₁-C₅ alkyl, C₂-C₅ alkenyl, C₂-C₅ alkynyl and C₁-C₅ alkylamino group is optionally substituted with hydroxy and/or amino group;
R3 and R4 are independently hydrogen or halogen;
with the proviso that when X is nitrogen, then R2 is hydrogen or halogen;
in agriculture as cytokinin oxidase inhibitors in the production of crop plants.

7. Use of the compound of the general formula (Ia) for inhibiting stress in whole plants, plant organs, plant tissues and/or plant cells.

8. Use of the compound of the general formula (Ia) for stimulation of seed germination, seedling growth and plant growth and development.

9. Use of the compound of the general formula (Ia) in preparations intended for the cloning of plant cells, plant tissues, plant organs and plants.

10. Use of the compound of the general formula (Ia) for inhibiting senescence and degradation of chlorophyll and chloroplasts.

11. A preparation for treatment of plants, **characterized in that** it contains at least one compound of the general formula (I) as defined in any one of the preceding claims 1 to 5 or a salt thereof with an alkali metal, ammonia or an amine in the form of a racemate or an optically active isomer, or an acid addition salt thereof;
and at least one excipient selected from the group comprising solvents, solid carriers, surfactants, emulsifiers, dispersants, wetting agents, wetting agents, stabilizers, defoamers, preservatives, viscosities, binders, adhesives, and fertilizers.

## Patentansprüche

1. Verbindung der allgemeinen Formel (I), 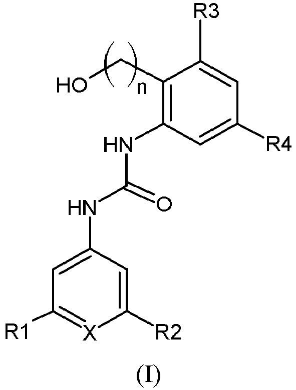 worin
n ist 1 oder 2;
X ist Stickstoff oder Kohlenstoff;
R1 ist ausgewählt aus der Gruppe bestehend aus Halogen, Methoxygruppe, Trifluormethoxygruppe, Methylsulfanylgruppe und Trifluormethylsulfanylgruppe;
R2 ist ausgewählt aus der Gruppe bestehend aus Wasserstoff, Halogen, Aminogruppe, C1-C5-Alkyl, C2-C5-Alkenyl, C2-C5-Alkinyl und C1-C5-Alkylaminogruppe, wobei die C1-C5-Alkyl, C2-C5-Alkenyl, C2-C5-Alkinyl und C1-C5-Alkylaminogruppe ist gegebenenfalls mit einer Hydroxy- und/oder Aminogruppe substituiert;
R3 und R4 sind unabhängig voneinander Wasserstoff oder Halogen;
mit der Maßgabe, dass, wenn X Stickstoff ist, R2 Wasserstoff oder Halogen ist;
und mit der Maßgabe, dass 1-(2,6-Dichlorpyridin-4-yl)-3-[2-(2-hydroxyethyl)phenyl]urea, 1-[2-(2-Hydroxyethyl)phenyl]-3-(3-(trifluormethoxy)phenyl)urea, 1-[2-(2-Hydroxyethyl)phenyl]-3-(3-methoxyphenyl)urea, 1-(3,5-Dichlorphenyl)-3-[2-(2-hydroxyethyl)phenyl]urea, 1-(3,5-Difluorphenyl)-3-[2-(2-hydroxyethyl)phenyl]urea, 1-(3-Fluorphenyl)-3-[2-(2-hydroxyethyl)phenyl]urea, 1-(3 -Chlorphenyl)-3 -[2-(2-hydroxyethyl)phenyl]urea, 1-(2-Hydroxymethylphenyl)-3-(3-methoxyphenyl)urea, 1-(3,5-Dichlorphenyl)-3-(2-hydroxymethylphenyl)urea, 1-(3-Fluorphenyl)-3-(2-hydroxymethylphenyl)urea und 1-(3-Chlorphenyl)-3-(2-hydroxymethylphenyl)urea sind ausgeschlossen.

2. Verbindung nach Anspruch 1, wobei R1 aus der Gruppe bestehend aus Fluor, Chlor, Brom, Methoxygruppe, Trifluormethoxygruppe, Methylsulfanylgruppe und Trifluormethylsulfanylgruppe ausgewählt ist.

3. Verbindung nach Anspruch 1 oder 2, wobei R2 aus der Gruppe bestehend aus Wasserstoff, Fluor, Chlor, Brom, Aminogruppe, 2-Aminoethylaminogruppe und 2-Hydroxyethylaminogruppe ausgewählt ist.

4. Verbindung nach Anspruch 1, 2 oder 3, wobei R3 und R4 unabhängig voneinander aus der Gruppe bestehend aus Wasserstoff, Fluor, Chlor und Brom ausgewählt sind.

5. Verbindung nach einem der vorhergehenden Ansprüche 1 bis 4, ausgewählt aus der Gruppe bestehend aus: 1-(2,6-Dichlorpyridin-4-yl)-3-(2-hydroxymethylphenyl)urea, 1-(5-Chlor)-2-hydroxymethylphenyl)-3-(2,6-dichlorpyridin-4-yl)urea, 1-(2-Methoxypyridin-4-yl)-3-(2-hydroxymethylphenyl)urea, 1-(5-Chlor-2-hydroxymethylphenyl)-3-(2-methoxypyridin-4-yl)urea, 1-(2-Hydroxymethylphenyl)-3-(3-trifluormethoxyphenyl)urea, 1-(5-Chlor-2-hydroxymethylphenyl)-3-(3-trifluormethoxyphenyl)urea, 1-(3-Chlor-5-trifluormethoxyphenyl)-3 -(2-hydroxymethylphenyl)urea, 1-(3 -Brom-5 -trifluormethoxyphenyl)-3 -(2-hydroxymethylphenyl)urea, 1-(3-Chlor-5-methoxyphenyl)-3-(2-hydroxymethylphenyl)urea, 1-(3-Brom-5-methoxyphenyl)-3-(2-hydroxymethylphenyl)urea, 1-(3-Brom-5-chlorphenyl)-3-(2-hydroxymethylphenyl)urea, 1-[2-(2-Hydroxyethyl)phenyl]-3-(3-trifluormethoxyphenyl)urea, 1-(3-Chlor-5-methoxyphenyl)-3-[2-(2-Hydroxyethyl)phenyl]urea, 1-(3-Brom-5-methoxyphenyl)-3-[2-(2-hydroxyethyl)phenyl]urea.

6. Verwendung einer Verbindung der allgemeinen Formel (Ia) 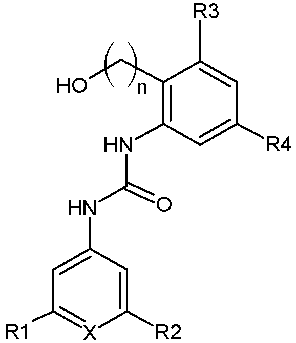 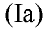 worin
n ist 1 oder 2;
X ist Stickstoff oder Kohlenstoff;
R1 ist ausgewählt aus der Gruppe bestehend aus Wasserstoff, Halogen, Methoxygruppe, Trifluormethoxygruppe, Methylsulfanylgruppe oder Trifluormethylsulfanylgruppe;
R2 ist ausgewählt aus der Gruppe bestehend aus Wasserstoff, Halogen, Aminogruppe, C1-C5-Alkyl, C2-C5-Alkenyl, C2-C5-Alkinyl und C1-C5-Alkylaminogruppe, wobei die C1-C5-Alkyl, C2-C5-Alkenyl, C2-C5-Alkinyl und C1-C5-Alkylaminogruppe ist gegebenenfalls mit einer Hydroxy- und/oder Aminogruppe substituiert;
R3 und R4 sind unabhängig voneinander Wasserstoff oder Halogen;
mit der Maßgabe, dass, wenn X Stickstoff ist, R2 Wasserstoff oder Halogen ist;
in der Landwirtschaft als Cytokininoxidase-Inhibitoren im Anbau von Kulturpflanzen.

7. Verwendung der Verbindung der allgemeinen Formel (Ia) zur Hemmung von Stress in ganzen Pflanzen, Pflanzenorganen, Pflanzengeweben und/oder Pflanzenzellen.

8. Verwendung der Verbindung der allgemeinen Formel (Ia) zur Stimulierung der Samenkeimung, des Keimlingswachstums sowie des Pflanzenwachstums und der Pflanzenentwicklung.

9. Verwendung der Verbindung der allgemeinen Formel (Ia) in Präparaten, die zum Klonen von Pflanzenzellen, Pflanzengeweben, Pflanzenorganen und Pflanzen bestimmt sind.

10. Verwendung der Verbindung der allgemeinen Formel (Ia) zur Hemmung der Seneszenz und des Abbaus von Chlorophyll und Chloroplasten.

11. Präparat zur Behandlung von Pflanzen, **dadurch gekennzeichnet, dass** es mindestens eine Verbindung der allgemeinen Formel (I) nach einem der vorhergehenden Ansprüche 1 bis 5 oder ein Salz davon mit einem Alkalimetall, Ammoniak oder einem Amin in Form eines Racemats oder eines optisch aktiven Isomers oder eines Säureadditionssalzes davon enthält;
und mindestens einen Hilfsstoff, ausgewählt aus der Gruppe bestehend aus Lösungsmitteln, festen Trägern, Tensiden, Emulgatoren, Dispergiermitteln, Netzmitteln, Netzmitteln, Stabilisatoren, Entschäumern, Konservierungsmitteln, Viskositätsmitteln, Bindemitteln, Klebstoffen und Düngemitteln.

## Revendications

1. Un composé de formule générale (I), 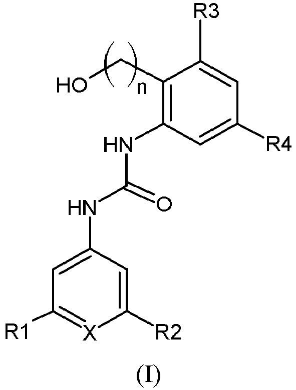 dans lequel
n est 1 ou 2;
X est l'azote ou le carbone;
R1 est choisi dans le groupe constitué d'un halogène, d'un groupe méthoxy, d'un groupe trifluorométhoxy, d'un groupe méthylsulfanyle et d'un groupe trifluorométhylsulfanyle;
R2 est choisi dans le groupe constitué d'un hydrogène, d'un halogène, d'un groupe amino, d'un (C₁ à C₅)alkyle, d'un (C₂ à C₅)alcényle, d'un (C₂ à C₅)alcynyle et d'un groupe (C₁ à C₅)alkylamino, dans lequel le group (C₁ à C₅)alkyle, (C₂ à C₅)alcényle, (C₂ à C₅)alcynyle et (C₁ à C₅)alkylamino est éventuellement substitué par un groupe hydroxy et/ou amino,
R3 et R4 sont indépendamment un hydrogène ou un halogène;
à condition que lorsque X est l'azote, alors R2 est l'hydrogène ou un halogène;
et à condition que 1-(2,6-dichloropyridin-4-yl)-3-[2-(2-hydroxyéthyl)phényl]urée, 1-[2-(2-hydroxyéthyl)phényl]-3-(3-(trifluorométhoxy)phényl)urée, 1-[2-(2-hydroxyéthyl)phényl]-3-(3-méthoxyphényl)urée, 1-(3,5-dichlorophényl)-3-[2-(2-hydroxyéthyl)phényl]urée, 1-(3,5-difluorophényl)-3-[2-(2-hydroxyéthyl)phényl]urée, 1-(3-fluorophényl)-3-[2-(2-hydroxyéthyl)phényl]urée, 1-(3 -chlorophényl)-3 -[2-(2-hydroxyéthyl)phényl]urée, 1-(2-hydroxyméthylphényl)-3 -(3 -méthoxyphényl)urée, 1-(3,5-dichlorophényl)-3-(2-hydroxyméthylphényl)urée, 1-(3-fluorophényl)-3-(2-hydroxyméthylphényl)urée et 1-(3-chlorophényl)-3-(2-hydroxyméthylphényl)urée sont exclues.

2. Le composé selon la revendication 1, dans lequel R1 est choisi dans le groupe constitué du fluor, du chlore, du brome, d'un groupe méthoxy, d'un groupe trifluorométhoxy, d'un groupe méthylsulfanyle et d'un groupe trifluorométhylsulfanyle.

3. Le composé selon la revendication 1 ou 2, dans lequel R2 est choisi dans le groupe constitué de l'hydrogène, du fluor, du chlore, du brome, d'un groupe amino, d'un groupe 2-aminoéthylamino et d'un groupe 2-hydroxyéthylamino.

4. Le composé selon la revendication 1, 2 ou 3, dans lequel R3 et R4 sont indépendamment choisis dans le groupe constitué de l'hydrogène, du fluor, du chlore et du brome.

5. Le composé selon l'une quelconque des revendications précédentes 1 à 4, choisi dans le groupe constitué de: 1-(2,6-dichloropyridin-4-yl)-3-(2-hydroxyméthylphényl)urée, 1-(5-chloro-2-hydroxyméthylphényl)-3-(2,6-dichloropyridin-4-yl)urée, 1-(2-méthoxypyridin-4-yl)-3-(2-hydroxyméthylphényl)urée, 1-(5-chloro-2-hydroxyméthylphényl)-3-(2-méthoxypyridin-4-yl)urée, 1-(2-hydroxyméthylphényl)-3-(3-trifluorométhoxyphényl)urée, 1-(5-chloro-2-hydroxyméthylphényl)-3-(3-trifluorométhoxyphényl)urée, 1-(3-chloro-5-trifluorométhoxyphényl)-3-(2-hydroxyméthylphényl)urée, 1-(3-bromo-5-trifluorométhoxyphényl)-3-(2-hydroxyméthylphényl)urée, 1-(3-chloro-5-méthoxyphényl)-3-(2-hydroxyméthylphényl)urée, 1-(3-bromo-5-méthoxyphényl)-3-(2-hydroxyméthylphényl)urée, 1-(3 -bromo-5 -chlorophényl)-3 -(2-hydroxyméthylphényl)urée, 1-[2-(2-hydroxyéthyl)phényl]-3-(3-trifluorométhoxyphényl)urée, 1-(3-chloro-5-méthoxyphényl)-3-[2-(2-hydroxyéthyl)phényl]urée, 1-(3-bromo-5-méthoxyphényl)-3-[2-(2-hydroxyéthyl)phényl]urée.

6. Utilisation du composé de formule générale (Ia) 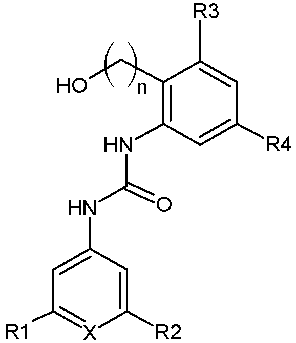 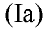 dans lequel
n est 1 ou 2;
X est l'azote ou le carbone;
R1 est choisi dans le groupe constitué d'un halogène, d'un groupe méthoxy, d'un groupe trifluorométhoxy, d'un groupe méthylsulfanyle et d'un groupe trifluorométhylsulfanyle;
R2 est choisi dans le groupe constitué d'un hydrogène, d'un halogène, d'un groupe amino, d'un (C₁ à C₅)alkyle, d'un (C₂ à C₅)alcényle, d'un (C₂ à C₅)alcynyle et d'un groupe (C₁ à C₅)alkylamino, dans lequel le group (C₁ à C₅)alkyle, (C₂ à C₅)alcényle, (C₂ à C₅)alcynyle et (C₁ à C₅)alkylamino est éventuellement substitué par un groupe hydroxy et/ou amino,
R3 et R4 sont indépendamment un hydrogène ou un halogène;
à condition que lorsque X est l'azote, alors R2 est l'hydrogène ou un halogène;
en agriculture comme inhibiteurs de la cytokinine oxydase dans la production de plantes cultivées.

7. Utilisation du composé de formule générale (Ia) pour inhiber le stress dans des plantes entières, des organes végétaux, des tissus végétaux et/ou des cellules végétales.

8. Utilisation du composé de formule générale (Ia) pour stimuler la germination des graines, la croissance des plantules ainsi que la croissance et le développement des plantes.

9. Utilisation du composé de formule générale (Ia) dans des préparations destinées au clonage de cellules végétales, de tissus végétaux, d'organes végétaux et de plantes.

10. Utilisation du composé de formule générale (Ia) pour inhiber la sénescence et la dégradation de la chlorophylle et des chloroplastes.

11. Préparation pour le traitement des plantes, **caractérisée en ce qu'**elle contient au moins un composé de formule générale (I) tel que défini dans l'une quelconque des revendications 1 à 5 précédentes ou un sel de celui-ci avec un métal alcalin, de l'ammoniaque ou une amine dans le forme d'un racémate ou d'un isomère optiquement actif, ou d'un sel d'addition acide de celui-ci; et au moins un excipient choisi dans le groupe comprenant les solvants, les supports solides, les tensioactifs, les émulsifiants, les dispersants, les agents mouillants, les stabilisants, les antimousses, les conservateurs, les viscosités, les liants, les adhésifs et les engrais.
